# EUROPEAN PATENT SPECIFICATION

(11) **EP 2 494 035 B1**
(45) Date of publication and mention of the grant of the patent: **28.02.2018**
(21) Application number: 10830437.9
(22) Date of filing: 28.10.2010
(51) Int. Cl.: C12N 5/074, C12N 5/02

(54) **PLURIPOTENT STEM CELLS**
PLURIPOTENTE STAMMZELLEN
CELLULES SOUCHES PLURIPOTENTES

(30) Priority: 29.10.2009 US 256149 P
(43) Date of publication of application: 05.09.2012
(73) Proprietor: Janssen Biotech, Inc., Horsham, PA 19044 (US)
(72) Inventor: FUNG, Ramie, Skillman, NJ 08558 (US); FRYER, Benjamin, Skillman, NJ 08558 (US)
(74) Representative: Carpmaels & Ransford LLP
(86) International application number: PCT/US2010/054408
(87) International publication number: WO 2011/059725

(56) References cited:
- EP-A1- 2 096 169
- WO-A1-2006/135824
- WO-A1-2009/061442
- WO-A1-2009/096049
- WO-A2-2004/055155
- WO-A2-2008/036447
- WO-A2-2008/118820
- WO-A2-2009/101407
- US-A1- 2009 203 141
- NING SUN ET AL: "Feeder-free derivation of induced pluripotent stem cells from adult human adipose stem cells", PROCEEDINGS OF THE NATIONAL ACADEMY OF SCIENCES, NATIONAL ACADEMY OF SCIENCES, US, vol. 106, no. 37, 15 September 2009 (2009-09-15), pages 15720-15725, XP008151219, ISSN: 0027-8424, DOI: 10.1073/PNAS.0908450106 [retrieved on 2009-09-08]
- LI C ET AL: "Pluripotency can be rapidly and efficiently induced in human amniotic fluid-derived cells", HUMAN MOLECULAR GENETICS, OXFORD UNIVERSITY PRESS, SURREY, vol. 18, no. 22, 13 August 2009 (2009-08-13), pages 4340-4349, XP002571841, ISSN: 0964-6906, DOI: 10.1093/HMG/DDP386 [retrieved on 2009-08-13]
- MEHDI TOTONCHI ET AL: "Feeder- and serum-free establishment and expansion of human induced pluripotent stem cells", THE INTERNATIONAL JOURNAL OF DEVELOPMENTAL BIOLOGY, vol. 54, no. 5, 2 October 2009 (2009-10-02), pages 877-886, XP055074858, ISSN: 0214-6282, DOI: 10.1387/ijdb.092903mt
- DONGHUI ZHANG ET AL: "Highly efficient differentiation of human ES cells and iPS cells into mature pancreatic insulin-producing cells", CELL RESEARCH, vol. 19, no. 4, 1 April 2009 (2009-04-01), pages 429-438, XP055074920, ISSN: 1001-0602, DOI: 10.1038/cr.2009.28
- Anonymous: "What are adult stem cells? [Stem Cell Information]", , 1 January 2015 (2015-01-01), XP055263534, Retrieved from the Internet: URL:http://stemcells.nih.gov/info/basics/p ages/basics4.aspx [retrieved on 2016-04-07]
- N. SUN ET AL: "Supplementary information for: "Feeder-free derivation of induced pluripotent stem cells from adult human adipose stem cells"", PROCEEDINGS OF THE NATIONAL ACADEMY OF SCIENCES, vol. 106, no. 37, 8 September 2009 (2009-09-08), pages 1-11, XP055319634, US ISSN: 0027-8424, DOI: 10.1073/pnas.0908450106

## Description

### FIELD OF THE INVENTION

The present invention provides methods to produce pluripotent stem cells from adult cells. In particular, the present invention provides methods to produce pluripotent stem cells from somatic cells without the use of a feeder-cell layer or an agent that increases efficiency of retroviral transfection.

### BACKGROUND

Advances in cell-replacement therapy for Type I diabetes mellitus and a shortage of transplantable islets of Langerhans have focused interest on developing sources of insulin-producing cells, or β cells, appropriate for engraftment. One approach is the generation of functional β cells from pluripotent stem cells, such as, for example, embryonic stem cells, or pluripotent stem cells generated from adult cells.

Pluripotent stem cells generated from adult cells, or "induced pluripotent stem cells", or "IPS cells" are a type of pluripotent stem cell artificially derived from a non-pluripotent cell, such as, for example an adult somatic cell, by inducing a "forced" expression of certain genes. Induced pluripotent stem cells are believed to be identical to natural pluripotent stem cells, such as embryonic stem cells in many respects, such as the expression of certain stem cell genes and proteins, chromatin methylation patterns, doubling time, embryoidbody formation, teratoma formation, viable chimera formation, and potency and differentiability.

In one example, Takahashi et al state "we demonstrate induction of pluripotent stem cells from mouse embryonic or adult fibroblasts by introducing four factors, Oct3/4, Sox2, c-Myc, and Klf4, under ES cell culture conditions." (Cell 126: 663-676, 2006).

In another example, Li et al state "we reveal combined genetic reprogramming and chemical conditions that generate and maintain rat iPSCs (riPSCs) that can give rise to teratomas and contribute extensively to chimeric rats. The same strategy is also sufficient to generate atypical human iPSCs (hiPSCs) that exhibit similar colony morphology and self-renewal requirements/signaling responses as those of mESCs." (Cell Stem Cell 4: 16-19, 2009).

In another example Maherali et al state "[e]ctopic expression of the four transcription factors Oct4, Sox2, c-Myc, and Klf4 is sufficient to confer a pluripotent state upon the fibroblast genome, generating induced pluripotent stem (iPS) cells. It remains unknown if nuclear reprogramming induced by these four factors globally resets epigenetic differences between differentiated and pluripotent cells. Here, using novel selection approaches, we have generated iPS cells from fibroblasts to characterize their epigenetic state. Female iPS cells showed reactivation of a somatically silenced X chromosome and underwent random X inactivation upon differentiation. Genome-wide analysis of two key histone modifications indicated that iPS cells are highly similar to ES cells. Consistent with these observations, iPS cells gave rise to viable high-degree chimeras with contribution to the germline. These data show that transcription factor-induced reprogramming leads to the global reversion of the somatic epigenome into an ES-like state." (Cell Stem Cell 1: 55-70,2007).

In another example, Stadtfeld et al state "[w]e have generated a doxycycline-inducible lentiviral system to transiently express the four reprogramming factors c-Myc, Klf4, Oct4, and Sox2 in fibroblasts." (Cell Stem Cell 2: 230-240).

In another example, Nakagawa et al state "[h]ere we describe a modified protocol for the generation of iPS cells that does not require the Myc retrovirus. With this protocol, we obtained significantly fewer non-iPS background cells, and the iPS cells generated were consistently of high quality. Mice derived from Myc- iPS cells did not develop tumors during the study period. The protocol also enabled efficient isolation of iPS cells without drug selection. Furthermore, we generated human iPS cells from adult dermal fibroblasts without MYC." (Nature Biotechnology 26: 101-106, 2008).

Ning Sun et al 2009 discloses feeder-free derivation of induced pluripotent stem cells from adult human adipose cells (PNAS 106; 37; 15720-15725).

In another example, Takahashi et al state "we demonstrate the generation of iPS cells from adult human dermal fibroblasts with the same four factors: Oct3/4, Sox2, Klf4, and c-Myc." (Cell 131: 861-872, 2007).

In another example, Okita et al state "[w]e have previously shown that pluripotent stem cells can be induced from mouse fibroblasts by retroviral introduction of Oct3/4 (also called Pou5fl), Sox2, c-Myc and Klf4, and subsequent selection for Fbx15 (also called Fbxo15) expression. These induced pluripotent stem (iPS) cells (hereafter called Fbx15 iPS cells) are similar to embryonic stem (ES) cells in morphology, proliferation and teratoma formation; however, they are different with regards to gene expression and DNA methylation patterns, and fail to produce adult chimaeras. Here we show that selection for Nanog expression results in germline-competent iPS cells with increased ES-cell-like gene expression and DNA methylation patterns compared with Fbx15 iPS cells." (Nature 448: 313-317, 2007).

In another example, Wernig et al state "fibroblasts can be reprogrammed to a pluripotent state by Oct4, Sox2, and Klf4 in the absence of c-Myc." (Cell Stem Cell 2: 10-12, 2008).

In another example, Park et al state "we have derived iPS cells from fetal, neonatal and adult human primary cells, including dermal fibroblasts isolated from a skin biopsy of a healthy research subject." (Nature 451: 141-146, 2008).

In another example, Yu et al state "We show that four factors (OCT4, SOX2, NANOG, and LIN28) are sufficient to reprogram human somatic cells to pluripotent stem cells that exhibit the essential characteristics of embryonic stem (ES) cells." (Science 318: 1917-1920, 2007).

However, this promise has not yet been fully tapped due, in part, to the difficulty in routinely deriving, passaging, and maintaining pluripotent stem cells derived from somatic cells in a pluripotent state. One characteristic limitation to the stable and consistent long term culture of pluripotent stem cells derived from somatic cells is the requirement that the pluripotent stem cells be derived on a feeder layer of cells. Feeder cells, usually mitotically inactive fibroblasts, provide a source of incompletely defined culture components which support adhesion, growth and passage of pluripotent stem cells derived from somatic cells. However, due to the variables inherent in feeder cells, feeder layer culture presents an obstacle to standardization of culture conditions and also to the directed differentiation of pluripotent stem cells derived from somatic cells. As a result, pluripotent stem cells derived from somatic cells are usually converted from feeder based culture to feeder free culture on an adlayer composed of extra cellular matrix protein(s) for more fully characterized and controlled culture and differentiation. Unfortunately, the process of converting pluripotent stem cells derived from somatic cells from culture on feeder cells to a feeder free system stresses the cells and can result in spontaneous differentiation and/or karyotypic instability

Therefore, there still remains a significant need to generate induced pluripotent stem cells of sufficient quality and that can be expanded to address the current clinical needs. The present invention utilizes an alternative approach to produce induced pluripotent stem cells, wherein pluripotent stem cells are formed from somatic cells without the use of a feeder cell layer.

### SUMMARY

In one embodiment, the present invention provides a method to produce pluripotent stem cells from somatic cells without the use of a feeder-cell layer or an agent that increases efficiency of viral transfection according to the claims.

In particular the invention provides a method to produce pluripotent stem cells from human amniotic fluid-derived cells without the use of a feeder-cell layer and without the use of an agent that increases efficiency of retroviral transfection comprising;
a) plating human amniotic fluid-derived cells on a tissue culture substrate;
b) transducing the plated amniotic fluid-derived cells with at least one retrovirus containing nucleic acid encoding SOX2, OCT4, LIN28, and NANOG;
c) culturing the transduced cells, then transferring the transduced cells onto tissue culture plates coated with an extracellular matrix protein, and culturing the transferred cells in medium supplemented with an agent that inhibits Rho kinase activity, and bFGF, for one week; and
d) passaging the cultured cells onto tissue culture plates coated with an extracellular matrix protein and culturing the passaged cells in conditioned medium containing soluble factors derived from feeder cells and supplemented with bFGF, wherein the cells are transduced to introduce SOX2, OCT4, LIN28 and NANOG into the cells.

The invention also provides a method to produce cells which are differentiated, for example cells expressing markers characteristic of the definitive endoderm lineage and/or cells expressing markers characteristic of the pancreatic endoderm lineage and/or cells expressing markers characteristic of the pancreatic endocrine lineage using a method according to the claims.

### BRIEF DESCRIPTION OF THE DRAWINGS

Figure 1 shows the expression of genes associated with pluripotency in cells of the human embryonic stem cell line H1 and pluripotent stem cells derived from the CRL2522 cell line.
Figure 2 shows the morphology of pluripotent stem cell colonies. Panels A and B show representative micrographs ofpluripotent stem cell colonies that were derived from amniotic fluid-derived cells. Panels C and D show pluripotent stem cells derived from amniotic fluid-derived cells (AFD1) after five passages. Panel E shows untransduced amniotic fluid-derived cells.
Figure 3 shows the expression of genes associated with pluripotency in pluripotent stem cells derived from amniotic fluid-derived cells.
Figure 4 shows the expression of genes associated with pluripotency in pluripotent stem cells derived from amniotic fluid-derived cells (AFD1), as detected via flow cytometry at the passage numbers indicated.
Figure 5 shows the expression of genes associated with pluripotency in pluripotent stem cells derived from amniotic fluid-derived cells (AFD1), as detected via immunofluorescence at passage 5.
Figure 6 shows the expression of genes associated with pluripotency in various pluripotent stem cells derived from the CRL2522 cell line.
Figure 7 shows the expression of markers characteristic of the definitive endoderm lineage in two populations of pluripotent stem cells derived from amniotic fluid-derived cells.
Figure 8 shows the expression of markers characteristic of the definitive endoderm lineage in a population of pluripotent stem cells derived from the CRL2522 cell line.
Figure 9 shows the expression of markers characteristic of the pancreatic endocrine lineage in a population of pluripotent stem cells derived from amniotic fluid-derived cells.
Figure 10 shows embryoid bodies formed using pluripotent stem cells derived from amniotic fluid-derived cells, CRL2522 cells and cells of the human embryonic stem cell line H1. A comparison of the gene expression profile of the various embryoid bodies is also shown.
Figure 11 shows the growth and attachment of pluripotent stem cells derived from amniotic fluid-derived cells to a surface that lacks a feeder-cell layer and an adlayer.
Figure 12 shows the expression of genes associated with pluripotency in pluripotent stem cells derived from amniotic fluid-derived cells that have been cultured on a surface that lacks a feeder-cell layer and an adlayer.
Figure 13 shows the expression of markers characteristic of the definitive endoderm lineage in a populations of pluripotent stem cells derived from amniotic fluid-derived cells that have been cultured on a surface that lacks a feeder-cell layer and an adlayer.

### DETAILED DESCRIPTION

For clarity of disclosure, and not by way of limitation, the detailed description of the invention is divided into the following subsections that describe or illustrate certain features, embodiments or applications of the present invention.

### Definitions

Stem cells are undifferentiated cells defined by their ability at the single cell level to both self-renew and differentiate to produce progeny cells, including self-renewing progenitors, non-renewing progenitors, and terminally differentiated cells. Stem cells are also characterized by their ability to differentiate *in vitro* into functional cells of various cell lineages from multiple germ layers (endoderm, mesoderm and ectoderm), as well as to give rise to tissues of multiple germ layers following transplantation and to contribute substantially to most, if not all, tissues following injection into blastocysts.

Stem cells are classified by their developmental potential as: (1) totipotent, meaning able to give rise to all embryonic and extraembryonic cell types; (2) pluripotent, meaning able to give rise to all embryonic cell types; (3) multipotent, meaning able to give rise to a subset of cell lineages but all within a particular tissue, organ, or physiological system (for example, hematopoietic stem cells (HSC) can produce progeny that include HSC (self- renewal), blood cell restricted oligopotent progenitors, and all cell types and elements (e.g., platelets) that are normal components of the blood); (4) oligopotent, meaning able to give rise to a more restricted subset of cell lineages than multipotent stem cells; and (5) unipotent, meaning able to give rise to a single cell lineage (e.g., spermatogenic stem cells).

Differentiation is the process by which an unspecialized ("uncommitted") or less specialized cell acquires the features of a specialized cell such as, for example, a nerve cell or a muscle cell. A differentiated or differentiation-induced cell is one that has taken on a more specialized ("committed") position within the lineage of a cell. The term "committed", when applied to the process of differentiation, refers to a cell that has proceeded in the differentiation pathway to a point where, under normal circumstances, it will continue to differentiate into a specific cell type or subset of cell types, and cannot, under normal circumstances, differentiate into a different cell type or revert to a less differentiated cell type. De-differentiation refers to the process by which a cell reverts to a less specialized (or committed) position within the lineage of a cell. As used herein, the lineage of a cell defines the heredity of the cell, i.e., which cells it came from and what cells it can give rise to. The lineage of a cell places the cell within a hereditary scheme of development and differentiation. A lineage-specific marker refers to a characteristic specifically associated with the phenotype of cells of a lineage of interest and can be used to assess the differentiation of an uncommitted cell to the lineage of interest.

"Cells expressing markers characteristic of the definitive endoderm lineage", or "Stage 1 cells", or "Stage 1", as used herein, refers to cells expressing at least one of the following markers: SOX17, GATA4, HNF3 beta, GSC, CER1, Nodal, FGF8, Brachyury, Mix-like homeobox protein, FGF4 CD48, eomesodermin (EOMES), DKK4, FGF17, GATA6, CXCR4, C-Kit, CD99, or OTX2. Cells expressing markers characteristic of the definitive endoderm lineage include primitive streak precursor cells, primitive streak cells, mesendoderm cells and definitive endoderm cells.

"Cells expressing markers characteristic of the pancreatic endoderm lineage", as used herein, refers to cells expressing at least one of the following markers: PDX1, HNF1 beta, PTF1 alpha, HNF6, NKX6.1, or HB9. Cells expressing markers characteristic of the pancreatic endoderm lineage include pancreatic endoderm cells, primitive gut tube cells, and posterior foregut cells.

"Definitive endoderm", as used herein, refers to cells which bear the characteristics of cells arising from the epiblast during gastrulation and which form the gastrointestinal tract and its derivatives. Definitive endoderm cells express the following markers: HNF3 beta, GATA4, SOX17, Cerberus, OTX2, goosecoid, C-Kit, CD99, and MIXL1.

"Markers", as used herein, are nucleic acid or polypeptide molecules that are differentially expressed in a cell of interest. In this context, differential expression means an increased level for a positive marker and a decreased level for a negative marker. The detectable level of the marker nucleic acid or polypeptide is sufficiently higher or lower in the cells of interest compared to other cells, such that the cell of interest can be identified and distinguished from other cells using any of a variety of methods known in the art.

"Pancreatic endocrine cell", or "pancreatic hormone expressing cell", as used herein, refers to a cell capable of expressing at least one of the following hormones: insulin, glucagon, somatostatin, and pancreatic polypeptide.

### Generation of the Induced Pluripotent Stem Cells

In one embodiment, the pluripotent stem cells of the present invention are derived from somatic cells without the use of a feeder-cell layer by introducing at least the genes SOX2, OCT4, LIN28 and NANOG according to the claims. Thee genes are introduced into human amniotic fluid-derived cells via viral transduction according to the claims.

In the case where the genes are introduced by viral transduction, the human amniotic fluid-derived cells are transduced using a retrovirus. Any retrovirus capable of introducing the genes into human amniotic fluid-derived cells is suitable for use in the present invention. In one embodiment, the retrovirus is a lentivirus.

In an alternate disclosure, the somatic cells may be transduced using a virus. Any virus capable of introducing the at least one gene into the somatic cell is suitable for use in the present invention. For example, the virus used in the methods of the present invention may be an adenovirus. Alternatively, the virus used in the methods of the present invention may be a baculovirus.

Agents that increase the efficiency of viral or retroviral transfection act by neutralizing the charge repulsion between virions and sialic acid on the cell membrane of the somatic cells. Such agents include, for example polybrene.

The at least one retrovirus may contain nucleic acid encoding one, or more than one of the at least one genes. In one embodiment, the human amniotic fluid-derived cells are transduced using retroviruses that contain nucleic acid encoding each gene singly.

In one embodiment, the conditioned medium is conditioned using mouse embryonic fibroblasts.

In one embodiment, the plated human amniotic fluid-derived cells are transduced with lentivirus containing nucleic acid encoding SOX2, OCT4, LIN28 and NANOG. In one embodiment, the plated human amniotic fluid-derived cells are transduced with at least one lentivirus containing nucleic acid encoding SOX2, OCT4, LIN28 and NANOG singly.

In one embodiment, the agent that inhibits Rho kinase activity is selected from the group consisting of: Y-27632, Fasudil, (S)-(+)-4-Glycyl-2-methyl-1-[(4-methyl-5-isoquinolinyl) sulfonyl]-hexahydro-1H-1,4-diazepine dihydrochloride (referred to herein as H1152-glycyl) and Hydroxyfasudil.

The extracellular matrix may be, for example fibronectin, vitronectin, laminin, collagen, gelatin, thrombospondin, and the like. In one embodiment, the extracellular matrix is MATRIGEL.

In one embodiment, the somatic cells may be the amniotic fluid-derived cells disclosed in US Patent Application 11/420,895, assigned to LifeScan, Inc.

In alternate embodiment, the somatic cells may be the amniotic fluid-derived cells disclosed in WO2003/042405.

In alternate embodiment, the somatic cells may be the amniotic fluid-derived cells disclosed in US Published Patent Application US 2005/0054093.

In alternate embodiment, the somatic cells may be the amniotic fluid-derived cells disclosed in Int' Anker et al, Blood 102: 1548-1549, 2003.

In alternate embodiment, the somatic cells may be the amniotic fluid-derived cells disclosed in Tsai et al, Human Reproduction 19, 1450-1456, 2004.

In an alternate embodiment, the somatic cells may be the chorionic villus-derived cells disclosed in US Patent Application 11/762,714, assigned to LifeScan, Inc.

In an alternate embodiment, the somatic cells may be the chorionic villus-derived cells disclosed in Chang & Jones, Prenatal Diagnosis 8: 367-378, 1988.

In an alternate embodiment, the somatic cells may be the chorionic villus-derived cells disclosed in Rong-Hao et al, Human Reproduction 11: 1328-1333, 1996.

In an alternate embodiment, the somatic cells may be the chorionic villus-derived cells disclosed in WO2003/042405.

In an alternate embodiment, the somatic cells may be the pancreatic-derived cells disclosed in WO2006/094286, assigned to LifeScan, Inc.

In an alternate embodiment, pluripotent stem cells may be formed from the cells disclosed in US Patent Application 12/108,872, assigned to LifeScan, Inc.

In one disclosure, conditioned medium is generated by a method which essentially involves:
a. Culturing cells that supply conditioning factors,
b. Adding a base medium to the cells,
c. Exposing the base medium to the cells for a period of time sufficient to condition the medium, and
d. Removing the conditioned medium.

In one disclosure, conditioned medium is generated by a method which essentially involves:
a. Culturing cells that supply conditioning factors,
b. Inactivating the cells,
c. Replating the cells that supply conditioning factors,
d. Adding a base medium to the cells,
e. Exposing the base medium to the cells for a period of time sufficient to condition the medium,
f. Removing the conditioned medium, and
g. Removing the conditioned medium.

"Base medium" as used herein refers to a solution of salts and nutrients that is effective to support the growth of non-pluripotent cells in culture.

"Conditioned medium" as used herein refers to a base medium that is further supplemented with soluble factors derived from feeder cells.

"Feeder cells" as used herein refers to non-pluripotent stem cells on which pluripotent stem cells are plated. The non-pluripotent stem cells provide a milieu conducive to the growth of the plated pluripotent stem cells.

The base medium used for conditioning can have any of several different formulae. The medium must be able to support the propagation of at least the cell line used for the conditioning of the medium. It is convenient that the medium also support the propagation of the pluripotent stem cells after conditioning. However, as an alternative, the medium can be supplemented with other factors or otherwise processed after conditioning to adapt it for propagating the pluripotent stem cells.

For propagating pluripotent stem cells in feeder-free culture, suitable base media can be made, from the following components, such as, for example, Dulbecco's modified Eagle's medium (DMEM), Gibco # 11965-092; Knockout Dulbecco's modified Eagle's medium (KO DMEM), Gibco # 10829-018; Ham's F12/50% DMEM basal medium; 200 mM L-glutamine, Gibco # 15039-027; non-essential amino acid solution, Gibco 11140-050; β-mercaptoethanol, Sigma # M7522; human recombinant basic fibroblast growth factor (bFGF), Gibco # 13256-029.

The base medium is combined with the cells used to condition the medium in an environment that allows the cells to release into the medium the components that propagate pluripotent stem cells. Optionally, the cells used to condition the medium can be inactivated (i.e., rendered incapable of substantial replication) by, for example, radiation, treatment with a chemical inactivator, such as, for example, mitomycin c, or by any other effective method. The inactivation of cells is not necessary in instances where the medium is separated from the conditioning cells before use in supporting pluripotent stem cell cultures.

The cells used to condition the medium are cultured in the medium for sufficient time to allow adequate concentration of released factors (or consumption of media components) to produce a medium that supports the propagation of pluripotent stem cells without differentiation. Typically, medium conditioned by culturing for 24 h at 37° C produces medium that supports pluripotent stem cell culture for 24 hours. However, the culturing period can be adjusted upwards or downwards, determining empirically (or by assaying for the concentration of essential factors) what constitutes an adequate period. After collecting a batch of conditioned medium, the cells can be used to condition a further batch of medium over a further culture period, for as many cycles as desired as long as the cells retain their ability to condition the medium in an adequate fashion.

### Expansion and Culture of the Induced Pluripotent Stem Cells

Pluripotent stem cells may express one or more of the stage-specific embryonic antigens (SSEA) 3 and 4, and markers detectable using antibodies designated Tra-1-60 and Tra-1-81 (Thomson et al., Science 282:1145, 1998). Differentiation of pluripotent stem cells *in vitro* results in the loss of SSEA-4, Tra 1-60, and Tra 1-81 expression (if present) and increased expression of SSEA-1. Undifferentiated pluripotent stem cells typically have alkaline phosphatase activity, which can be detected by fixing the cells with 4% paraformaldehyde, and then developing with Vector Red as a substrate, as described by the manufacturer (Vector Laboratories, Burlingame Calif.). Undifferentiated pluripotent stem cells also typically express OCT4 and TERT, as detected by RT-PCR.

Another desirable phenotype of propagated pluripotent stem cells is a potential to differentiate into cells of all three germinal layers: endoderm, mesoderm, and ectoderm tissues. Pluripotency of pluripotent stem cells can be confirmed, for example, by injecting cells into severe combined immunodeficient (SCID) mice, fixing the teratomas that form using 4% paraformaldehyde, and then examining them histologically for evidence of cell types from the three germ layers. Alternatively, pluripotency may be determined by the creation of embryoid bodies and assessing the embryoid bodies for the presence of markers associated with the three germinal layers.

Propagated pluripotent stem cell lines may be karyotyped using a standard G-banding technique and compared to published karyotypes of the corresponding primate species. It is desirable to obtain cells that have a "normal karyotype," which means that the cells are euploid, wherein all human chromosomes are present and not noticeably altered.

### Culture of Pluripotent Stem Cells

Pluripotent stem cells formed by the methods of the present disclosure are cultured on a layer of feeder cells that support the pluripotent stem cells in various ways. Pluripotent stem cells formed by the methods of the present invention are cultured in a culture system that is essentially free of feeder cells, but nonetheless supports proliferation of pluripotent stem cells without undergoing substantial differentiation. The growth of pluripotent stem cells formed by the methods of the present invention in feeder-free culture without differentiation is supported using a medium conditioned by culturing previously with another cell type. Alternatively, the growth of pluripotent stem cells formed by the methods of the present invention in feeder-free culture without differentiation is supported using a chemically defined medium.

For example, Reubinoff et al (Nature Biotechnology 18: 399 - 404 (2000)) and Thompson et al (Science 6 November 1998: Vol. 282. no. 5391, pp. 1145 - 1147) disclose the culture of pluripotent stem cell lines from human blastocysts using a mouse embryonic fibroblast feeder cell layer.

Richards et al, (Stem Cells 21: 546-556, 2003) evaluated a panel of 11 different human adult, fetal and neonatal feeder cell layers for their ability to support human pluripotent stem cell culture. Richards *et al*, states: "human embryonic stem cell lines cultured on adult skin fibroblast feeders retain human embryonic stem cell morphology and remain pluripotent".

US20020072117 discloses cell lines that produce media that support the growth of primate pluripotent stem cells in feeder-free culture. The cell lines employed are mesenchymal and fibroblast-like cell lines obtained from embryonic tissue or differentiated from embryonic stem cells. US20020072117 also discloses the use of the cell lines as a primary feeder cell layer.

In another example, Wang et al (Stem Cells 23: 1221-1227, 2005) discloses methods for the long-term growth of human pluripotent stem cells on feeder cell layers derived from human embryonic stem cells.

In another example, Stojkovic et al (Stem Cells 2005 23: 306-314,2005) disclose a feeder cell system derived from the spontaneous differentiation of human embryonic stem cells.

In a further example, Miyamoto et al (Stem Cells 22: 433-440, 2004) disclose a source of feeder cells obtained from human placenta.

Amit et al (Biol. Reprod 68: 2150-2156, 2003) discloses a feeder cell layer derived from human foreskin.

In another example, Inzunza et al (Stem Cells 23: 544-549, 2005) disclose a feeder cell layer from human postnatal foreskin fibroblasts.

US6642048 discloses media that support the growth of primate pluripotent stem (pPS) cells in feeder-free culture, and cell lines useful for production of such media. US6642048 states: "This invention includes mesenchymal and fibroblast-like cell lines obtained from embryonic tissue or differentiated from embryonic stem cells. Methods for deriving such cell lines, processing media, and growing stem cells using the conditioned media are described and illustrated in this disclosure."

In another example, WO2005014799 discloses conditioned medium for the maintenance, proliferation and differentiation of mammalian cells. WO2005014799 states: "The culture medium produced in accordance with the present invention is conditioned by the cell secretion activity of murine cells; in particular, those differentiated and immortalized transgenic hepatocytes, named MMH (Met Murine Hepatocyte)."

In another example, Xu et al (Stem Cells 22: 972-980, 2004) discloses conditioned medium obtained from human embryonic stem cell derivatives that have been genetically modified to over express human telomerase reverse transcriptase.

In another example, US20070010011 discloses a chemically defined culture medium for the maintenance of pluripotent stem cells.

An alternative culture system employs serum-free medium supplemented with growth factors capable of promoting the proliferation of embryonic stem cells. For example, Cheon et al (BioReprod DOI:10.1095/biolreprod.105.046870, October 19, 2005) disclose a feeder-free, serum-free culture system in which embryonic stem cells are maintained in unconditioned serum replacement (SR) medium supplemented with different growth factors capable of triggering embryonic stem cell self-renewal.

In another example, Levenstein et al (Stem Cells 24: 568-574, 2006) disclose methods for the long-term culture of human embryonic stem cells in the absence of fibroblasts or conditioned medium, using media supplemented with bFGF.

In another example, US20050148070 discloses a method of culturing human embryonic stem cells in defined media without serum and without fibroblast feeder cells, the method comprising: culturing the stem cells in a culture medium containing albumin, amino acids, vitamins, minerals, at least one transferrin or transferrin substitute, at least one insulin or insulin substitute, the culture medium essentially free of mammalian fetal serum and containing at least about 100 ng/ml of a fibroblast growth factor capable of activating a fibroblast growth factor signaling receptor, wherein the growth factor is supplied from a source other than just a fibroblast feeder layer, the medium supported the proliferation of stem cells in an undifferentiated state without feeder cells or conditioned medium.

In another example, US20050233446 discloses a defined media useful in culturing stem cells, including undifferentiated primate primordial stem cells. In solution, the media is substantially isotonic as compared to the stem cells being cultured. In a given culture, the particular medium comprises a base medium and an amount of each of bFGF, insulin, and ascorbic acid necessary to support substantially undifferentiated growth of the primordial stem cells.

In another example, US6800480 states "In one embodiment, a cell culture medium for growing primate-derived primordial stem cells in a substantially undifferentiated state is provided which includes a low osmotic pressure, low endotoxin basic medium that is effective to support the growth of primate-derived primordial stem cells. The basic medium is combined with a nutrient serum effective to support the growth of primate-derived primordial stem cells and a substrate selected from the group consisting of feeder cells and an extracellular matrix component derived from feeder cells. The medium further includes non-essential amino acids, an anti-oxidant, and a first growth factor selected from the group consisting of nucleosides and a pyruvate salt."

In another example, US20050244962 states: "In one aspect the invention provides a method of culturing primate embryonic stem cells. One cultures the stem cells in a culture essentially free of mammalian fetal serum (preferably also essentially free of any animal serum) and in the presence of fibroblast growth factor that is supplied from a source other than just a fibroblast feeder layer. In a preferred form, the fibroblast feeder layer, previously required to sustain a stem cell culture, is rendered unnecessary by the addition of sufficient fibroblast growth factor."

In a further example, WO2005065354 discloses a defined, isotonic culture medium that is essentially feeder-free and serum-free, comprising: a. a basal medium; b. an amount of bFGF sufficient to support growth of substantially undifferentiated mammalian stem cells; c. an amount of insulin sufficient to support growth of substantially undifferentiated mammalian stem cells; and d. an amount of ascorbic acid sufficient to support growth of substantially undifferentiated mammalian stem cells.

In another example, WO2005086845 discloses a method for maintenance of an undifferentiated stem cell, said method comprising exposing a stem cell to a member of the transforming growth factor-beta (TGF-β) family of proteins, a member of the fibroblast growth factor (FGF) family of proteins, or nicotinamide (NIC) in an amount sufficient to maintain the cell in an undifferentiated state for a sufficient amount of time to achieve a desired result.

The pluripotent stem cells formed by the methods of the present invention may be plated onto a suitable culture substrate. In one embodiment, the suitable culture substrate is an extracellular matrix component, such as, for example, those derived from basement membrane or that may form part of adhesion molecule receptor-ligand couplings. In one embodiment, the suitable culture substrate is MATRIGEL® (Becton Dickenson). MATRIGEL® is a soluble preparation from Engelbreth-Holm Swarm tumor cells that gels at room temperature to form a reconstituted basement membrane.

Other extracellular matrix components and component mixtures are suitable as an alternative. Depending on the cell type being proliferated, this may include laminin, fibronectin, proteoglycan, entactin, heparan sulfate, and the like, alone or in various combinations.

The pluripotent stem cells formed by the methods of the present invention may be plated onto the substrate in a suitable distribution and in the presence of a medium that promotes cell survival, propagation, and retention of the desirable characteristics. All these characteristics benefit from careful attention to the seeding distribution and can readily be determined by one of skill in the art.

Suitable culture media may be made from the following components, such as, for example, Dulbecco's modified Eagle's medium (DMEM), Gibco # 11965-092; Knockout Dulbecco's modified Eagle's medium (KO DMEM), Gibco #10829-018; Ham's F12/50% DMEM basal medium; 200 mM L-glutamine, Gibco # 15039-027; non-essential amino acid solution, Gibco 11140-050; β-mercaptoethanol, Sigma # M7522; human recombinant basic fibroblast growth factor (bFGF), Gibco # 13256-029.

### Differentiating Pluripotent Stem Cells Generated Using the Methods of the Present Invention

Pluripotent stem cells formed using the methods of the present invention may be differentiated into a variety of other cell types by any suitable method in the art. For example, the pluripotent stem cells formed using the methods of the present invention may be differentiated into neural cells, cardiac cells, hepatocytes, pancreatic cells, and the like.

For example, pluripotent stem cells formed using the methods of the present invention may be differentiated into neural progenitors and cardiomyocytes according to the methods disclosed in WO2007030870.

In another example, pluripotent stem cells formed using the methods of the present invention may be differentiated into hepatocytes according to the methods disclosed in US patent 6,458,589.

### Differentiation of Pluripotent Stem Cells Formed Using the Methods of the Present Intention into Cells Expressing Markers Characteristic of the Definitive Endoderm Lineage

Pluripotent stem cells formed using the methods of the present invention may be differentiated into cells expressing markers characteristic of the definitive endoderm lineage by any method in the art.

For example, pluripotent stem cells formed using the methods of the present invention may be differentiated into cells expressing markers characteristic of the definitive endoderm lineage according to the methods disclosed in D'Amour et al, Nature Biotechnology 23, 1534 - 1541 (2005).

For example, pluripotent stem cells formed using the methods of the present invention may be differentiated into cells expressing markers characteristic of the definitive endoderm lineage according to the methods disclosed in Shinozaki et al, Development 131, 1651 - 1662 (2004).

For example, pluripotent stem cells formed using the methods of the present invention may be differentiated into cells expressing markers characteristic of the definitive endoderm lineage according to the methods disclosed in McLean et al, Stem Cells 25, 29 - 38 (2007).

For example, pluripotent stem cells formed using the methods of the present invention may be differentiated into cells expressing markers characteristic of the definitive endoderm lineage according to the methods disclosed in D'Amour et al, Nature Biotechnology 24, 1392 - 1401 (2006).

In another example, pluripotent stem cells formed using the methods of the present invention may be differentiated into cells expressing markers characteristic of the definitive endoderm lineage according to the methods disclosed in US patent application Ser. No. 11/736,908, assigned to LifeScan, Inc.

In another example, pluripotent stem cells formed using the methods of the present invention may be differentiated into cells expressing markers characteristic of the definitive endoderm lineage according to the methods disclosed in US patent application Ser. No. 11/779,311, assigned to LifeScan, Inc.

In another example, pluripotent stem cells formed using the methods of the present invention may be differentiated into cells expressing markers characteristic of the definitive endoderm lineage according to the methods disclosed in US patent application Ser. No. 12/493,741, assigned to LifeScan, Inc.

In another example, pluripotent stem cells formed using the methods of the present invention may be differentiated into cells expressing markers characteristic of the definitive endoderm lineage according to the methods disclosed in US patent application Ser. No. 12/494,789, assigned to LifeScan, Inc.

Formation of cells expressing markers characteristic of the definitive endoderm lineage may be determined by testing for the presence of the markers before and after following a particular protocol. Pluripotent stem cells typically do not express such markers. Thus, differentiation of pluripotent cells is detected when cells begin to express them.

### Differentiation of Pluripotent Stem Cells Formed Using the Methods of the Present Invention into Cells Expressing Markers Characteristic of the Pancreatic Endoderm Lineage

Pluripotent stem cells formed using the methods of the present invention may be differentiated into cells expressing markers characteristic of the pancreatic endoderm lineage by any method in the art.

For example, pluripotent stem cells may be differentiated into cells expressing markers characteristic of the pancreatic endoderm lineage according to the methods disclosed in D'Amour et al, Nature Biotechnology 24, 1392 - 1401 (2006).

For example, cells expressing markers characteristic of the definitive endoderm lineage obtained according to the methods of the present invention are further differentiated into cells expressing markers characteristic of the pancreatic endoderm lineage, by treating the cells expressing markers characteristic of the definitive endoderm lineage with a fibroblast growth factor and the hedgehog signaling pathway inhibitor KAAD-cyclopamine, then removing the medium containing the fibroblast growth factor and KAAD-cyclopamine and subsequently culturing the cells in medium containing retinoic acid, a fibroblast growth factor and KAAD-cyclopamine. An example of this method is disclosed in Nature Biotechnology 24, 1392 - 1401 (2006).

For example, cells expressing markers characteristic of the definitive endoderm lineage obtained according to the methods of the present invention are further differentiated into cells expressing markers characteristic of the pancreatic endoderm lineage, by treating the cells expressing markers characteristic of the definitive endoderm lineage with retinoic acid one fibroblast growth factor for a period of time, according to the methods disclosed in US patent application Ser. No. 11/736,908, assigned to LifeScan, Inc.

For example, cells expressing markers characteristic of the definitive endoderm lineage obtained according to the methods of the present invention are further differentiated into cells expressing markers characteristic of the pancreatic endoderm lineage, by treating the cells expressing markers characteristic of the definitive endoderm lineage with retinoic acid (Sigma-Aldrich, MO) and exendin 4, then removing the medium containing DAPT (Sigma-Aldrich, MO) and exendin 4 and subsequently culturing the cells in medium containing exendin 1, IGF-1 and HGF. An example of this method is disclosed in Nature Biotechnology 24, 1392 - 1401 (2006).

For example, cells expressing markers characteristic of the pancreatic endoderm lineage obtained according to the methods of the present invention are further differentiated into cells expressing markers characteristic of the pancreatic endocrine lineage, by culturing the cells expressing markers characteristic of the pancreatic endoderm lineage in medium containing exendin 4, then removing the medium containing exendin 4 and subsequently culturing the cells in medium containing exendin 1, IGF-1 and HGF. An example of this method is disclosed in D'Amour et al, Nature Biotechnology, 2006.

For example, cells expressing markers characteristic of the pancreatic endoderm lineage obtained according to the methods of the present invention are further differentiated into cells expressing markers characteristic of the pancreatic endocrine lineage, by culturing the cells expressing markers characteristic of the pancreatic endoderm lineage in medium containing DAPT (Sigma-Aldrich, MO) and exendin 4. An example of this method is disclosed in D'Amour et al, Nature Biotechnology, 2006.

For example, cells expressing markers characteristic of the pancreatic endoderm lineage obtained according to the methods of the present invention are further differentiated into cells expressing markers characteristic of the pancreatic endocrine lineage, by culturing the cells expressing markers characteristic of the pancreatic endoderm lineage in medium containing exendin 4. An example of this method is disclosed in D'Amour et al, Nature Biotechnology, 2006.

For example, cells expressing markers characteristic of the pancreatic endoderm lineage obtained according to the methods of the present invention are further differentiated into cells expressing markers characteristic of the pancreatic endocrine lineage, by treating the cells expressing markers characteristic of the pancreatic endoderm lineage with a factor that inhibits the Notch signaling pathway, according to the methods disclosed in US patent application Ser. No. 11/736,908, assigned to LifeScan, Inc.

For example, cells expressing markers characteristic of the pancreatic endoderm lineage obtained according to the methods of the present invention are further differentiated into cells expressing markers characteristic of the pancreatic endocrine lineage, by treating the cells expressing markers characteristic of the pancreatic endoderm lineage with a factor that inhibits the Notch signaling pathway, according to the methods disclosed in US patent application Ser. No. 11/779,311, assigned to LifeScan, Inc.

For example, cells expressing markers characteristic of the pancreatic endoderm lineage obtained according to the methods of the present invention are further differentiated into cells expressing markers characteristic of the pancreatic endocrine lineage, by treating the cells expressing markers characteristic of the pancreatic endoderm lineage with a factor that inhibits the Notch signaling pathway, according to the methods disclosed in US patent application Ser. No. 60/953,178, assigned to LifeScan, Inc.

For example, cells expressing markers characteristic of the pancreatic endoderm lineage obtained according to the methods of the present invention are further differentiated into cells expressing markers characteristic of the pancreatic endocrine lineage, by treating the cells expressing markers characteristic of the pancreatic endoderm lineage with a factor that inhibits the Notch signaling pathway, according to the methods disclosed in US patent application Ser. No. 60/990,529, assigned to LifeScan, Inc.

Markers characteristic of the definitive endoderm lineage are selected from the group consisting of SOX17, GATA4, HNF3 beta, GSC, CER1, Nodal, FGF8, Brachyury, Mix-like homeobox protein, FGF4 CD48, eomesodermin (EOMES), DKK4, FGF17, GATA6, CXCR4, C-Kit, CD99, and OTX2. Suitable for use in the present invention is a cell that expresses at least one of the markers characteristic of the definitive endoderm lineage. In one aspect of the present invention, a cell expressing markers characteristic of the definitive endoderm lineage is a primitive streak precursor cell. In an alternate aspect, a cell expressing markers characteristic of the definitive endoderm lineage is a mesendoderm cell. In an alternate aspect, a cell expressing markers characteristic of the definitive endoderm lineage is a definitive endoderm cell.

Markers characteristic of the pancreatic endoderm lineage are selected from the group consisting of PDX1, HNF1 beta, PTF1 alpha, HNF6, HB9 and PROX1. Suitable for use in the present invention is a cell that expresses at least one of the markers characteristic of the pancreatic endoderm lineage. In one aspect of the present invention, a cell expressing markers characteristic of the pancreatic endoderm lineage is a pancreatic endoderm cell.

### Differentiation of Pluripotent Stem Cells Formed Using the Methods of the Present Invention into Cells Expressing Markers Characteristic of the Pancreatic Endocrine Lineage

Pluripotent stem cells formed using the methods of the present invention may be differentiated into cells expressing markers characteristic of the pancreatic endocrine lineage by any method in the art.

For example, cells expressing markers characteristic of the pancreatic endoderm lineage obtained according to the methods of the present invention are further differentiated into cells expressing markers characteristic of the pancreatic endocrine lineage, by culturing the cells expressing markers characteristic of the pancreatic endoderm lineage in medium containing exendin 4, then removing the medium containing exendin 4 and subsequently culturing the cells in medium containing exendin 1, IGF-1 and HGF. An example of this method is disclosed in D'Amour et al, Nature Biotechnology, 2006.

For example, cells expressing markers characteristic of the pancreatic endoderm lineage obtained according to the methods of the present invention are further differentiated into cells expressing markers characteristic of the pancreatic endocrine lineage, by culturing the cells expressing markers characteristic of the pancreatic endoderm lineage in medium containing DAPT (Sigma-Aldrich, MO) and exendin 4. An example of this method is disclosed in D'Amour et al, Nature Biotechnology, 2006.

For example, cells expressing markers characteristic of the pancreatic endoderm lineage obtained according to the methods of the present invention are further differentiated into cells expressing markers characteristic of the pancreatic endocrine lineage, by culturing the cells expressing markers characteristic of the pancreatic endoderm lineage in medium containing exendin 4. An example of this method is disclosed in D'Amour et al, Nature Biotechnology, 2006.

For example, cells expressing markers characteristic of the pancreatic endoderm lineage obtained according to the methods of the present invention are further differentiated into cells expressing markers characteristic of the pancreatic endocrine lineage, by treating the cells expressing markers characteristic of the pancreatic endoderm lineage with a factor that inhibits the Notch signaling pathway, according to the methods disclosed in US patent application Ser. No. 11/736,908, assigned to LifeScan, Inc.

For example, cells expressing markers characteristic of the pancreatic endoderm lineage obtained according to the methods of the present invention are further differentiated into cells expressing markers characteristic of the pancreatic endocrine lineage, by treating the cells expressing markers characteristic of the pancreatic endoderm lineage with a factor that inhibits the Notch signaling pathway, according to the methods disclosed in US patent application Ser. No. 11/779,311, assigned to LifeScan, Inc.

For example, cells expressing markers characteristic of the pancreatic endoderm lineage obtained according to the methods of the present invention are further differentiated into cells expressing markers characteristic of the pancreatic endocrine lineage, by treating the cells expressing markers characteristic of the pancreatic endoderm lineage with a factor that inhibits the Notch signaling pathway, according to the methods disclosed in US patent application Ser. No. 60/953,178, assigned to LifeScan, Inc.

For example, cells expressing markers characteristic of the pancreatic endoderm lineage obtained according to the methods of the present invention are further differentiated into cells expressing markers characteristic of the pancreatic endocrine lineage, by treating the cells expressing markers characteristic of the pancreatic endoderm lineage with a factor that inhibits the Notch signaling pathway, according to the methods disclosed in US patent application Ser. No. 60/990,529, assigned to LifeScan, Inc.

Markers characteristic of the pancreatic endocrine lineage are selected from the group consisting of NGN3, NEUROD, ISL1, PDX1, NKX6.1, PAX4, and PTF-1 alpha. In one embodiment, a pancreatic endocrine cell is capable of expressing at least one of the following hormones: insulin, glucagon, somatostatin, and pancreatic polypeptide. Suitable for use in the present invention is a cell that expresses at least one of the markers characteristic of the pancreatic endocrine lineage. In one aspect of the present invention, a cell expressing markers characteristic of the pancreatic endocrine lineage is a pancreatic endocrine cell. The pancreatic endocrine cell may be a pancreatic hormone-expressing cell. Alternatively, the pancreatic endocrine cell may be a pancreatic hormone-secreting cell.

In one aspect of the present invention, the pancreatic endocrine cell is a cell expressing markers characteristic of the β cell lineage. A cell expressing markers characteristic of the β cell lineage expresses PDX1 and at least one of the following transcription factors: NGN3, NKX2.2, NKX6.1, NEUROD, ISL1, HNF3 beta, MAFA, PAX4, and PAX6. In one aspect of the present invention, a cell expressing markers characteristic of the β cell lineage is a β cell.

### Therapies

Disclosed herein are methods for treating a patient suffering from, or at risk of developing, Type1 diabetes. The method may involve generating pluripotent stem cells from somatic cells, culturing the pluripotent stem cells, differentiating the pluripotent stem cells *in vitro* into a β-cell lineage, and implanting the cells of a β-cell lineage into a patient.

If appropriate, the patient can be further treated with pharmaceutical agents or bioactives that facilitate the survival and function of the transplanted cells. These agents may include, for example, insulin, members of the TGF-β family, including TGF-β1, 2, and 3, bone morphogenic proteins (BMP-2, -3, -4, -5, -6, -7, -11, -12, and -13), fibroblast growth factors-1 and -2, platelet-derived growth factor-AA, and -BB, platelet rich plasma, insulin growth factor (IGF-I, II) growth differentiation factor (GDF-5, -6, -7, -8, - 10, -15), vascular endothelial cell-derived growth factor (VEGF), pleiotrophin, endothelin, among others. Other pharmaceutical compounds can include, for example, nicotinamide, glucagon like peptide-I (GLP-1) and II, GLP-1 and 2 mimetibody, Exendin-4, retinoic acid, parathyroid hormone, MAPK inhibitors, such as, for example, compounds disclosed in U.S. Published Application 2004/0209901 and U.S. Published Application 2004/0132729.

The pluripotent stem cells generated from somatic cells may be differentiated into an insulin-producing cell prior to transplantation into a recipient. The pluripotent stem cells generated from somatic cells may be fully differentiated into β-cells, prior to transplantation into a recipient. Alternatively, the pluripotent stem cells may be transplanted into a recipient in an undifferentiated or partially differentiated state. Further differentiation may take place in the recipient.

Definitive endoderm cells or, alternatively, pancreatic endoderm cells, or, alternatively, β cells, may be implanted as dispersed cells or formed into clusters that may be infused into the hepatic portal vein. Alternatively, cells may be provided in biocompatible degradable polymeric supports, porous non-degradable devices or encapsulated to protect from host immune response. Cells may be implanted into an appropriate site in a recipient. The implantation sites include, for example, the liver, natural pancreas, renal subcapsular space, omentum, peritoneum, subserosal space, intestine, stomach, or a subcutaneous pocket.

To enhance further differentiation, survival or activity of the implanted cells, additional factors, such as growth factors, antioxidants or anti-inflammatory agents, can be administered before, simultaneously with, or after the administration of the cells. Growth factors may be utilized to differentiate the administered cells *in vivo.* These factors can be secreted by endogenous cells and exposed to the administered cells *in situ.* Implanted cells can be induced to differentiate by any combination of endogenous and exogenously administered growth factors known in the art.

The amount of cells used in implantation depends on a number of various factors including the patient's condition and response to the therapy, and can be determined by one skilled in the art.

Disclosed herein are methods for treating a patient suffering from, or at risk of developing diabetes. This method involves culturing pluripotent stem cells, differentiating the cultured cells *in vitro* into a β-cell lineage, and incorporating the cells into a three-dimensional support. The cells can be maintained *in vitro* on this support prior to implantation into the patient. Alternatively, the support containing the cells can be directly implanted in the patient without additional *in vitro* culturing. The support can optionally be incorporated with at least one pharmaceutical agent that facilitates the survival and function of the transplanted cells.

Support materials suitable for use for purposes of the present invention include tissue templates, conduits, barriers, and reservoirs useful for tissue repair. In particular, synthetic and natural materials in the form of foams, sponges, gels, hydrogels, textiles, and nonwoven structures, which have been used *in vitro* and *in vivo* to reconstruct or regenerate biological tissue, as well as to deliver chemotactic agents for inducing tissue growth, are suitable for use in practicing the methods of the present invention. See, for example, the materials disclosed in U.S. Patent 5,770,417, U.S. Patent 6,022,743, U.S. Patent 5,567,612, U.S. Patent 5,759,830, U.S. Patent 6,626,950, U.S. Patent 6,534,084, U.S. Patent 6,306,424, U.S. Patent 6,365,149, U.S. Patent 6,599,323, U.S. Patent 6,656,488, U.S. Published Application 2004/0062753 A1, U.S. Patent 4,557,264and U.S. Patent 6,333,029.

To form a support incorporated with a pharmaceutical agent, the pharmaceutical agent can be mixed with the polymer solution prior to forming the support. Alternatively, a pharmaceutical agent could be coated onto a fabricated support, preferably in the presence of a pharmaceutical carrier. The pharmaceutical agent may be present as a liquid, a finely divided solid, or any other appropriate physical form. Alternatively, excipients may be added to the support to alter the release rate of the pharmaceutical agent. The support can be incorporated with at least one pharmaceutical compound that is an anti-inflammatory compound, such as, for example compounds disclosed in U.S. Patent 6,509,369.

The support may be incorporated with at least one pharmaceutical compound that is an anti-apoptotic compound, such as, for example, compounds disclosed in U.S. Patent 6,793,945.

The support may also be incorporated with at least one pharmaceutical compound that is an inhibitor of fibrosis, such as, for example, compounds disclosed in U.S. Patent 6,331,298.

The support may also be incorporated with at least one pharmaceutical compound that is capable of enhancing angiogenesis, such as, for example, compounds disclosed in U.S. Published Application 2004/0220393 and U.S. Published Application 2004/0209901.

The support may also be incorporated with at least one pharmaceutical compound that is an immunosuppressive compound, such as, for example, compounds disclosed in U.S. Published Application 2004/0171623.

The support may also be incorporated with at least one pharmaceutical compound that is a growth factor, such as, for example, members of the TGF-β family, including TGF-β1, 2, and 3, bone morphogenic proteins (BMP-2, -3,-4, -5, -6, -7, -11, -12, and -13), fibroblast growth factors-1 and -2, platelet-derived growth factor-AA, and -BB, platelet rich plasma, insulin growth factor (IGF-I, II) growth differentiation factor (GDF-5, -6, -8, -10, -15), vascular endothelial cell-derived growth factor (VEGF), pleiotrophin, endothelin, among others. Other pharmaceutical compounds can include, for example, nicotinamide, hypoxia inducible factor 1-alpha, glucagon like peptide-I (GLP-1), GLP-1 and GLP-2 mimetibody, and II, Exendin-4, nodal, noggin, NGF, retinoic acid, parathyroid hormone, tenascin-C, tropoelastin, thrombin-derived peptides, cathelicidins, defensins, laminin, biological peptides containing cell- and heparin-binding domains of adhesive extracellular matrix proteins such as fibronectin and vitronectin, MAPK inhibitors, such as, for example, compounds disclosed in U.S. Published Application 2004/0209901 and U.S. Published Application 2004/0132729.

The incorporation of the cells of the present invention into a scaffold can be achieved by the simple depositing of cells onto the scaffold. Cells can enter into the scaffold by simple diffusion (J. Pediatr. Surg. 23 (1 Pt 2): 3-9 (1988)). Several other approaches have been developed to enhance the efficiency of cell seeding. For example, spinner flasks have been used in seeding of chondrocytes onto polyglycolic acid scaffolds (Biotechnol. Prog. 14(2): 193-202 (1998)). Another approach for seeding cells is the use of centrifugation, which yields minimum stress to the seeded cells and enhances seeding efficiency. For example, Yang et al. developed a cell seeding method (J. Biomed. Mater. Res. 55(3): 379-86 (2001)), referred to as Centrifugational Cell Immobilization (CCI).

The present invention is further illustrated, but not limited by, the following examples.

### EXAMPLES

### Reference Example 1

### Generation of Pluripotent Stem Cells from Somatic Cells with Feeder Cells using the STEMGENT™ Human Transfection Factor Kit

Using the method stated in the Stemgent™ human transfection factor kit, pluripotent stem cells were generated from adult foreskin fibroblast cells, using the Stemgent™ Human TF Lentivirus Set (Cat. No. 00-0005). CRL2522 cells from ATCC (human foreskin fibroblasts referred to as "BJ" cells in the Stemgent protocol) were plated to a 6 well plate in growth media at a density of 100,000 cells/ well which resulted in a confluency of 40-50%. The growth media consisted of 450 ml EMEM, 50ml ES-qualified FBS, 5ml 10mM Non-Essential Amino Acids, 5 ml penicillin (10,000 U/ml)-streptomycin (10,000 µg/ml), 5 ml 200 mM L-glutamine, and 0.9 ml 55 mM β-mercaptoethanol (referred to herein as BJ cell growth media). The day after plating the cells, media was changed to fresh BJ cell growth media plus polybrene and lentiviruses in a total volume of 2.5ml (Conditions #1 and 2, Table 1). Lentivirus viral titer was determined by the manufacturer using p24 capsid antigen ELISA assay. Viral titers were as follows: SOX2-Lentivirus titer- 68.80ng/ml; OCT4-Lentivirus titer- 6.64ng/ml; LIN28-Lentivirus titer- 68.80ng/ml; andNANOG-Lentivirus titer- 82.80ng/ml.

After ensuring that the medium was distributed evenly by gentle rocking of the cell culture dish, the cells were incubated overnight at 37°C and 5% CO₂. Twenty four hours post-transduction, the cells were trypsinized, centrifuged at 200 x g for 5 minutes, resuspended in BJ cell growth medium, and re-plated in 3 wells of a 6-well at a 1 to 3 plating ratio onto CF-1 MEF feeder cells seeded the previous day. The virally transduced CRL2522 cells were incubated on the feeder cells overnight at 37°C and 5% CO₂. Twenty four hours after re-seeding, BJ cell growth medium was replaced with human ES/iPS cell culture medium (comprising DMEM-F12 Media- 200ml, Knockout Serum Replacer- 50ml, 200mM L-Glutamine + 2-Mercaptoethanol Solution- 1.25ml, Non-essential Amino Acids 100X Solution- 2.5ml, and B-FGF Solution- 4ng/ml final concentration). The ES/iPS cell culture medium was changed every day for the first seven days. After seven days, medium was replaced to MEF conditioned medium (MEFCM). MEFCM was generated by exposing ES/iPS cell culture medium to mouse embryonic fibroblasts.

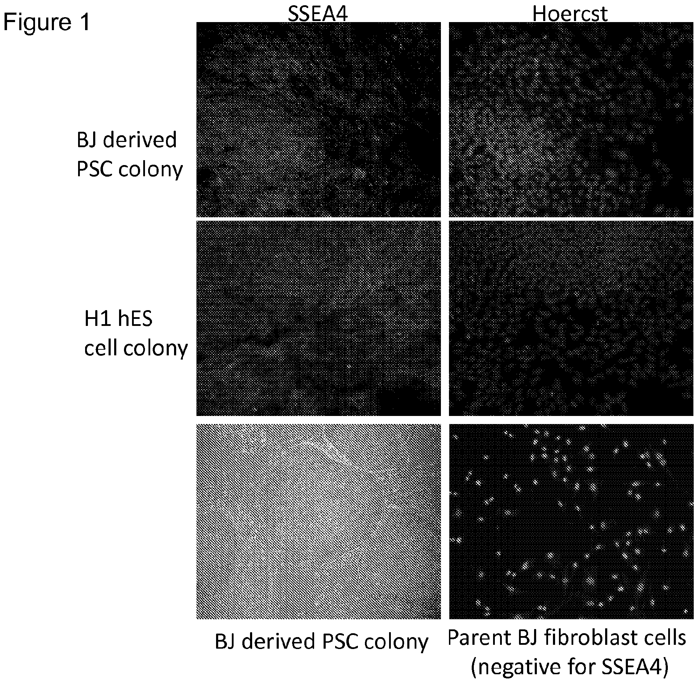

After thirty eight days in culture the cells were enzymatically passaged from with dispase to MATRIGEL™ in MEFCM supplemented with 10ng/ml bFGF and 5µM of the Rho kinase inhibitor Y27632. Thereafter, cells were fed daily with fresh MEFCM supplemented with 10ng/ml bFGF. 42 days after viral transduction the first small pluripotent colonies were observed. Passaging of cells continued until they showed typical human ES morphology. Twelve clonal lines were generated. Cell lines were banked and cryopreserved at passage 6, and 3 clones have now been passaged in culture 15 times. The clones maintain pluripotent morphology and the characteristics of pluripotent stem cells. Furthermore, the cells can be differentiated to all three germ layers using the embryoid body assay, and their differentiation can be specifically directed to form definitive endoderm using a defined protocol. Micrographs of typical pluripotent stem cell colonies are shown in Figure 1.

### Example 2

### Generation of Pluripotent Stem Cells from Somatic Cells using the STEMGENT™ Human Transfection Factor Kit Without the use of Feeder Cells

100,000 amniotic fluid-derived cells isolated according to the methods disclosed in US Patent Application 11/420,895 were plated in AMNIOMAX™ media in a 10cm² well of a six well dish. The following day cells were transduced with lentivirus virus according to Condition #7 in Table 1. Media was changed to fresh AMNIOMAX™ media plus lentiviruses in a total volume of 2.5ml. Lentivirus viral titer was determined by the manufacturer using p24 capsid antigen ELISA assay. Viral titers were as follows: SOX2-Lentivirus titer- 68.80ng/ml; OCT4-Lentivirus titer- 6.64ng/ml; LIN28-Lentivirus titer- 68.80ng/ml; and NANOG-Lentivirus titer- 82.80ng/ml.

The viral transduction used in this example did not use the transduction enhancement agent, polybrene (The following day cells were transduced with lentivirus virus according to Condition #7, Table 1). On day 3, cells were split and plated to a surface coated with a 1:30 dilution of MATRIGEL™ with MEFCM supplemented with 8ng/ml of bFGF (MEFCM8) and 5µM of the ROCK inhibitor Y27632 for passage zero (p0). Cells were plated and fed daily thereafter with fresh MEFCM8.

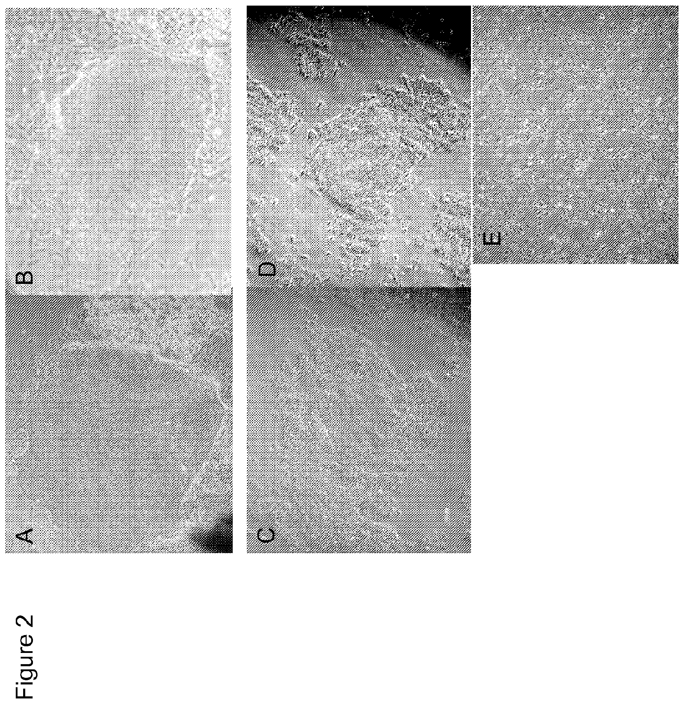

After one week in culture, cells were dispase passaged to fresh 1:30 MATRIGEL™ coated plates (first passage =p1). 10 days later approximately 20 human embryonic stem cell like colonies were observed (Figure 2, A&B). The most dense "Embryonic stem cell like" colonies were manually selected and combined for passage. This culture was designated cell line AFD1 (Figure 2, C&D). A single colony was manually selected for passage and designated AFD2.

Cell lines AFD1 and 2 were banked and cryopreserved at passage 5 and have now been passaged in culture 11-12 times and have maintained pluripotent morphology and the characteristics of pluripotent stem cells.

### Example 3

### Generation of Pluripotent Stem Cells from Somatic Cells using the STEMGENT™ Human Transfection Factor Kit Without the use of Feeder Cells

We were not able to derive pluripotent cells using a feeder layer of MEF cells without the use of polybrene on feeder cells (see Conditions #3-6, Table 1). CRL22429 cells (foreskin fibroblast cells, ATCC, Manassas VA USA) or AF cells were plated at a concentration of 100,000 cells into separate 10cm² wells of a six well dish overnight. The next day cells were treated with fresh media containing the recommended amount of virus for 24 hours without polybrene. On the third day cells were TrypLE treated and passaged onto MEF cells in hESC/iPS cell media with 5µM of the ROCK inhibitor Y27632 to promote adhesion. Parallel populations of cells were also treated with three compounds that have been reported to improve pluripotent stem cell colony formation: CHIR99021 at 100nM, BIX01294 at 1µM, and R (T)Bay K 8644 at 2µM. Media was changed each day for 14 days. Wells containing feeders and AF cells exhibited significant death after 14 days in culture.

In an attempt to rescue the cells, they were collagenase passaged onto a new feeder layer in hESC/iPS cell media without supplementary compounds. No colonies were observed after additional time in culture. Wells that contained CRL2429 and feeder cells remained viable after 6 weeks in culture. After 6 weeks in culture the cells were passaged onto 1:30 matrigel with collagenase, however no colonies were observed. Regardless of the addition of compound, significant cell death was observed in co-cultures of AF cells with feeders as compared to co-cultures with CRL2429 cells and feeders.

### Example 4

### Characterization of the Pluripotent Stem Cells Derived According to the Methods of the Present Invention

Pluripotent stem cells were derived according to the methods described in Examples 1 and 2. The pluripotent stem cells were characterized by qRT-PCR, immunoflorescence, flow cytometry, and phase contrast microscopy to confirm gene expression and morphology common to pluripotent stem cells.

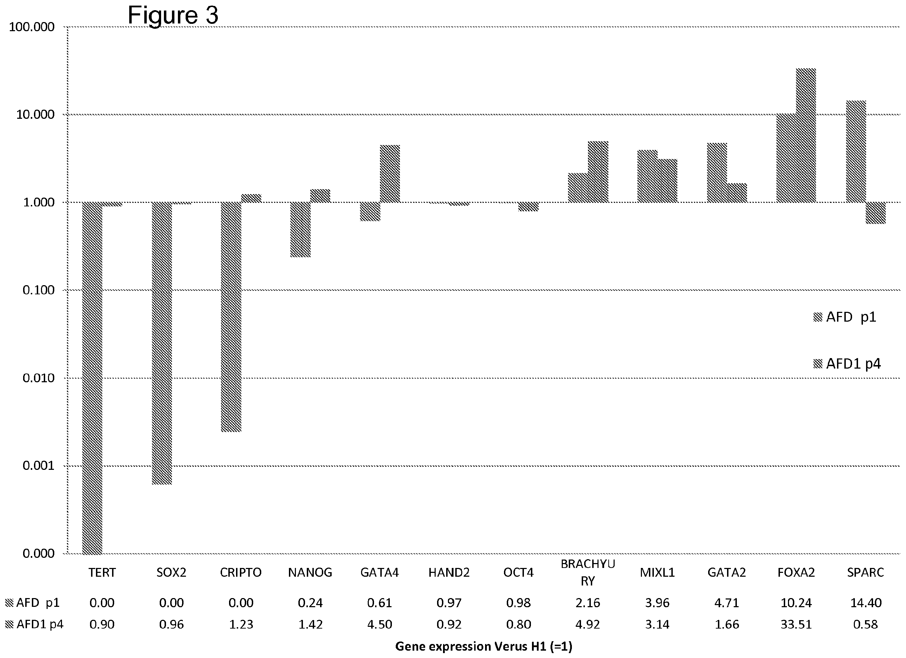

Using qRT-PCR (Figure 3) expression of genes characteristic of pluripotency (OCT4, NANOG, SOX2, TERT, and CRIPTO) in cell line AFD at passage 4 (p4) was observed at levels comparable to the human embryonic stem cell line H1 (set to an expression level of 1.0). It was also noted that the parent population of AF cells (AFD), 13 days after viral transduction at passage 1 (p1) but before appearance of pluripotent colonies of cells, did not have significant expression of SOX2, TERT, and CRIPTO, and had much lower expression of NANOG than AFD1 at p4. These results indicate that the expression of SOX2, and NANOG observed at p4 was due to endogenous expression, and not the viral construct which is consistent with published observations that full conversion of a somatic cell to a pluripotent state after viral transduction requires silencing of the viral construct. Interestingly, AFD expression of OCT4 at p1 was at levels comparable to the hESC line H1 and remained high at p4, suggesting that early and sustained OCT4 expression is required for adoption of a pluripotent state.

Similarly, pluripotent stem cells derived from CRL2522 cells also expressed genes characteristic of pluripotent cells: OCT4, NANOG, FGF4 and CDH1 were expressed at levels comparable to the human embryonic stem cell line H1 (set to an expression level of 1.0) and were expressed at levels significantly higher than expression levels observed in the parent CRL2522 cell line.

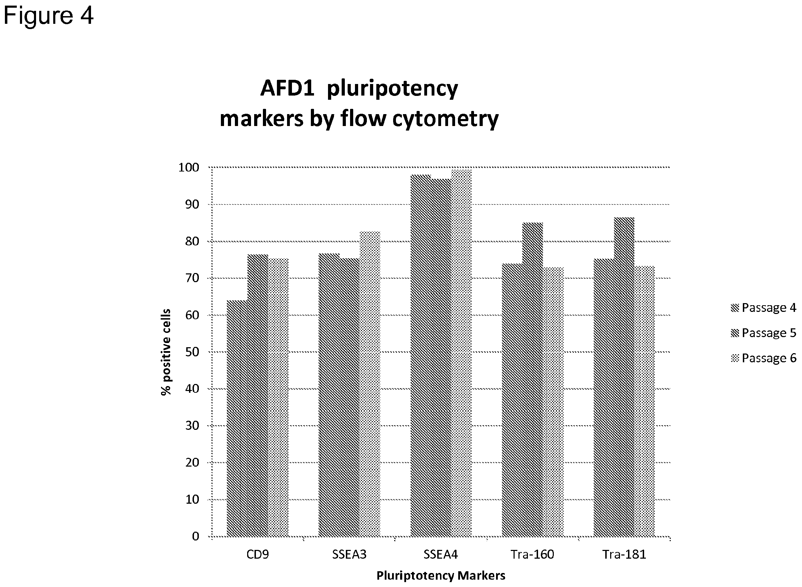

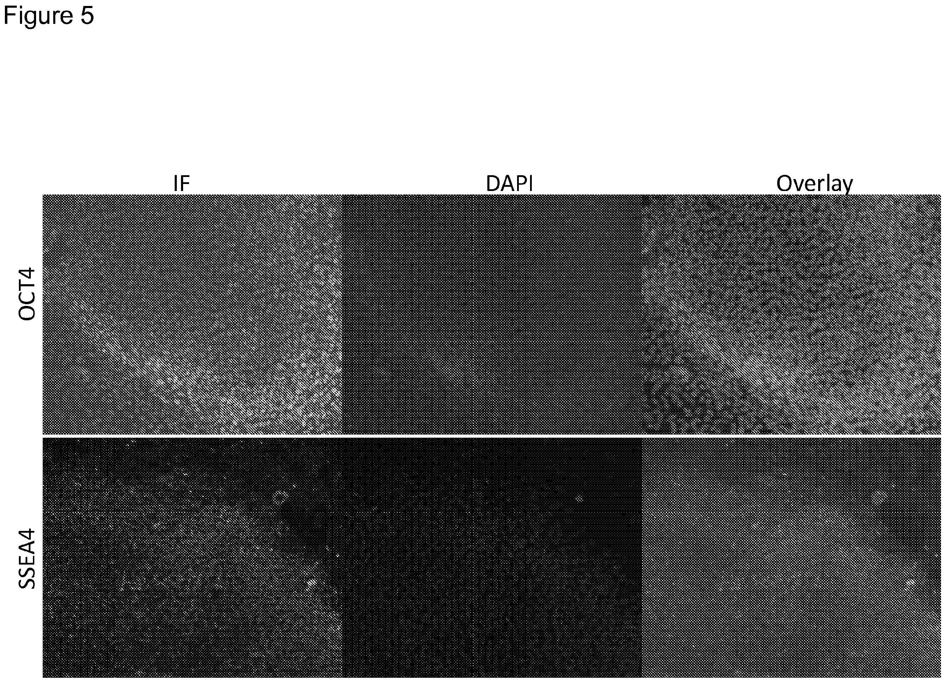

The cells were also characterized by phase microscopy (Figure 2) demonstrating that AFD1 pluripotent stem cell colonies had a dense center with smooth edges consistent with human embryonic stem cell colony appearance, and a significantly different morphology from the parent population of AF cells. When assayed by flow cytometry at passages 4, 5, and 6, we observed that AFD1 cells had high surface expression of markers characteristic of pluripotent stem cells, including SSEA3, SSEA4, CD9, TRA-160, and TRA-181 (Figure 4), proteins not expressed on the parent AF cells. Expression of SSEA4 by immunoflorescence was also observed, and the expression patterns were similar to those observed for OCT4 (Figure 5). These results were consistent with expression patterns found on the human embryonic stem cell line H1, and were also replicated with pluripotent stem cells derived from CRL2522 cells (Figure 1). Furthermore, the nuclear density of the cells was significantly higher than untransduced parent CRL2522 fibroblast cells, which were negative for SSEA4 expression (Figure 1).

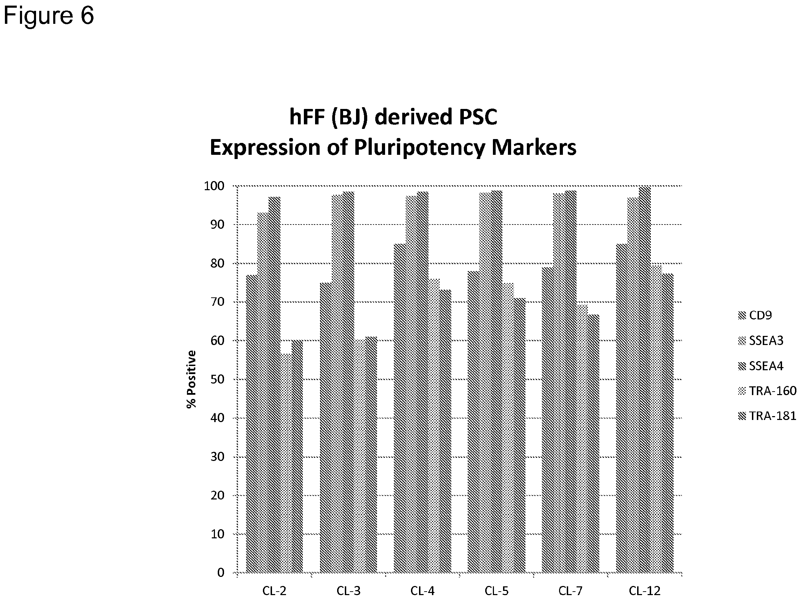

When assayed by flow cytometry at passage 6, pluripotent stem cells derived from CRL2522 cells had high surface expression of markers characteristic of pluripotent stem cells, including SSEA3, SSEA4, CD9, TRA-160, and TRA-181 (Figure 6), proteins not expressed on parent CRL2522 cells. Expression of SSEA4 by immunoflorescence was also observed, and the expression patterns were similar to those observed for OCT4 (Figure 1). These results were consistent with expression patterns found on the hESC line H1, and indicate that we derived pluripotent stem cells from human foreskin fibroblast CRL2522 "BJ" cells were derived (Figure 1).

### Example 5

### Differentiation of the Pluripotent Stem Cells Derived According to the Methods of the Present Invention

Pluripotent stem cells generated from human foreskin fibroblasts or amniotic fluid cells according to the methods of the present invention were differentiated using several different methods to confirm their pluripotency. Pluripotent stem cells were differentiated to cells expressing markers characteristic of the definitive endoderm lineage, and cells expressing markers characteristic of the pancreatic endoderm lineage. Additionally, the pluripotent stem cells produced by the methods of the present invention were shown to form embryoid bodies when placed into non-adherent suspension culture.

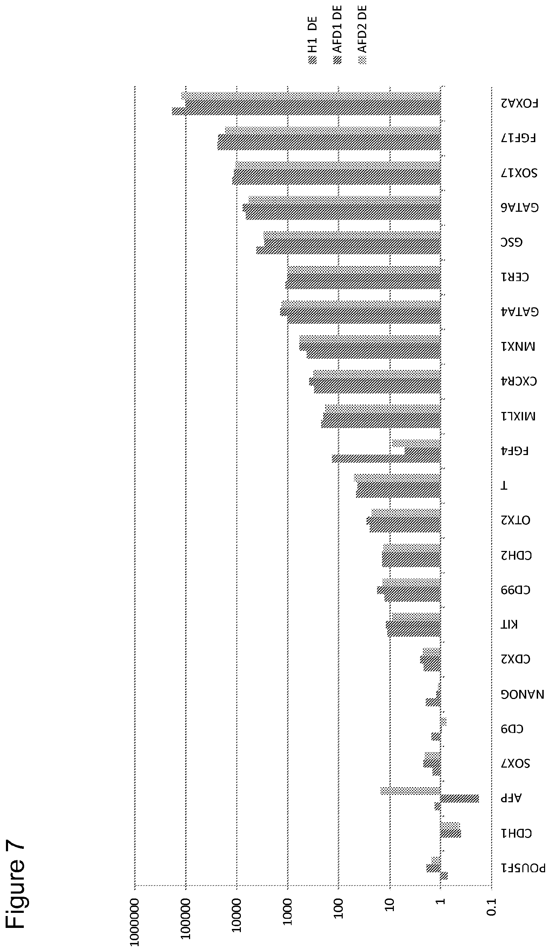

The pluripotent stem cells of the present invention were differentiated into cells expressing markers characteristic of the definitive endoderm lineage (DE) by treating the pluripotent stem cells with DE media, comprising RPMI 1640 media containing 2% fatty acid free bovine serum albumin (FAFBSA), 20ng/ml Wnt3a, 8ng/ml bFGF, and 100ng/ml Activin A. On day 2 and day 3 of differentiation, Wnt3a was removed from the DE media. After 3 days in culture samples were lifted by TrypLE™ treatment and assayed by flow cytometry for expression of the marker CD184 (the protein CXCR4) which correlates with formation of definitive endoderm. Amniotic Fluid Derived pluripotent stem cells 1 and 2 (AFD1 and AFD2) expressed similar levels of CXCR4 and CD99, when compared with H1 human embryonic stem cells when directed to differentiate to definitive endoderm. These results were confirmed by qRT-PCR, which showed a strikingly similar induction of genes characteristic of DE (CXCR4, SOX17, GSC, CER1, and FOXA2) versus the human ES line H1 (Figure 7).

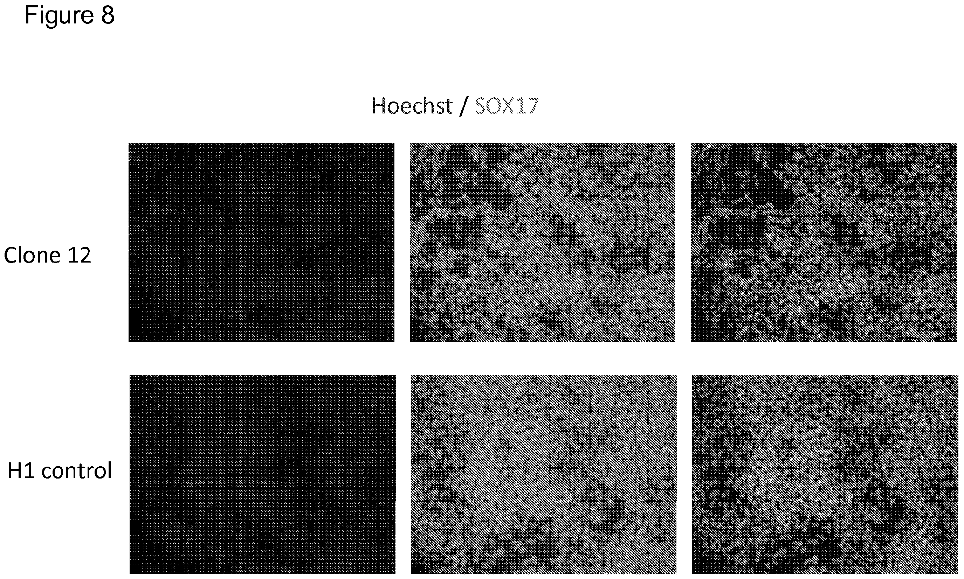

Similar results were observed with human foreskin fibroblast, CRL2522-derived pluripotent stem cells. 8 clones of CRL2522 derived pluripotent stem cells were differentiated to definitive endoderm, and each expressed elevated levels of the DE marker, CXCR4, with expression levels ranging from 23.4% to 58.3% positive versus an H1 hES cell control which was 60% positive for CXCR4 (Table 2). Samples of CRL2522 derived pluripotent stem cells differentiated to DE were also observed to express high levels as detected by immunoflorescence of SOX17, a transcription factor characteristic of and required for, definitive endoderm (Figure 8). These results indicate that AF and CRL2522 derived pluripotent stem cells form definitive endoderm, as measured by flow cytometry, qRT-PCR, and immunofluorescence, at a rate consistent with DE formation by the human embryonic stem cell line, H1.

In normal development, definitive endoderm contributes to multiple lineages including lung, gut, liver, pancreas, thymus, and thyroid. The differentiation from DE to cells expressing markers characteristic of the pancreatic endoderm lineage was directed using a protocol shown to promote a pancreatic fate in H1 human embryonic stem cells. The additional differentiation stages beyond the first stage of DE formation included: Stage **2-** RPMI 1640 media containing 2% FAF BSA, 0.25 µM Sant-1, and 50ng/ml FGF-7 for two days; Stage **3-** DMEM high glucose media containing 0.5X ITS, 0.1% BSA, 0.25uM Sant-1, 50ng/ml FGF-7,10ng/ml Noggin, 20 ng/ml Activin A, and 2 µM RA for four days; Stage **3.5-** DMEM high glucose media containing 0.5X ITS, 0.1% BSA, long/ml FGF-7, and 100ng/ml Noggin for 3 days; Stage **4**- DMEM high glucose containing 0.5X ITS, 0.1% BSA, long/ml FGF-7, 100ng/ml Noggin, and 1 µM ALK5 inhibitor for 3 days; and Stage **5-** DMEM high glucose media containing 0.5X ITS, 0.1% BSA, 100ng/ml Noggin, and 1 µM ALK5 inhibitor for 7 days.

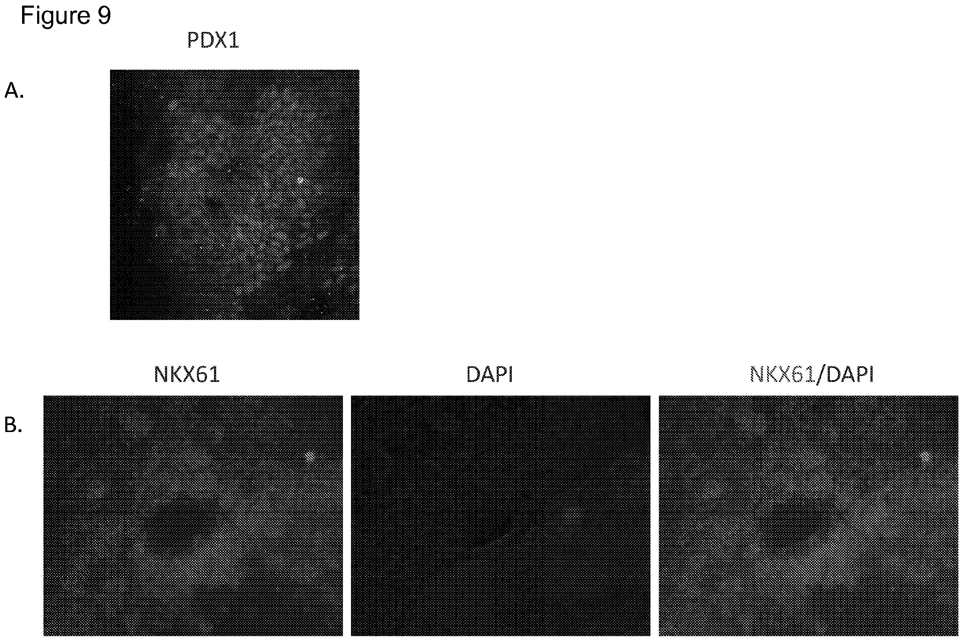

Using this method a significant increase in expression of the pancreatic hormones; insulin, glucagon, and somatostatin, and an increase in transcription factors common to pancreatic islet beta cells, including NKX6.1, PDX1, and NEUROD as measured by qRT-PCR in PSCs derived from both Amniotic Fluid and CRL2522 cells (Table 3) was observed. NKX6.1 expression and PDX1 expression in amniotic fluid derived pluripotent stem cells differentiated through Stage 5, as measured by immunoflorescence (Figure 9), was also observed. These results indicate that pluripotent stem cells derived from either AF or CRL2522 cells are competent to form the endoderm germ layer, and can be differentiated to a pancreatic fate.

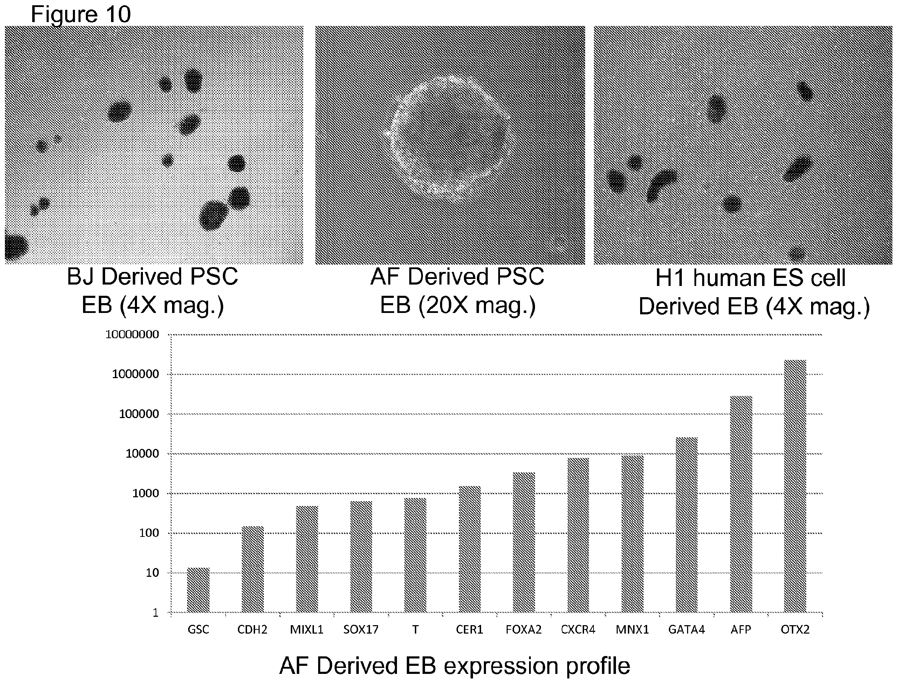

In addition to determining pluripotency of AF and CRL2522 derived pluripotent stem cells by directed differentiation, it was tested if the cells could form all three germ layers using the embryoidbody formation assay. Pluripotent stem cells derived from AF or CRL2522 cells were grown on a matrigel coated culture surface and were lifted from the surface by incubating the cells with a 0.1% dispase solution, washing the cells with DMEM media supplemented with 10% FBS, lifting the cells with a rubber scraper and then transferring the cells in DMEM media supplemented with 10% FBS to a low binding culture dish for non-adherent, suspension culture. Pluripotent stem cells grown in suspension culture in high serum containing media will spontaneously form embryoid bodies; spherical multi-cellular structures composed of all three germ layer lineages. The formation of the lineages is confirmed by qRT-PCR assay for gene expression common to each of the lineages (mesoderm, endoderm, and ectoderm). Pluripotent stem cells derived from either AF or CRL2522 cells formed embryoid bodies and had gene expression patterns indicating that all three germ layers were present (Figure 10).

Markers of neuronal/ectodermal lineages (CDH2 and OTX) were elevated, while early markers for mesendoderm (T) and definitive endoderm (SOX17, AFP, FOXA2, and CXCR4) were also elevated, as were markers common to mesoderm and definitive endoderm (GSC, CER1, GATA4 and MIXL1) and also a marker found in motor neurons and beta islet cells (MNX). (Figure 10). These results indicate that the pluripotent stem cells generated from AF or CRL2522 cells were indeed pluripotent and competent to generate tissues from each of the three germ layers.

### Example 6

### The Effects of Rho Kinase Inhibition on the Culture of the Pluripotent Stem Cells of the Present Invention, when the Cells are Cultured in the Absence of feeder cells or Extracellular Matrix Proteins

The pluripotent stem cell line AFD1, was maintained in MEF conditioned media on Nunclon Delta™ plates treated with a 1:30 dilution of growth factor reduced MATRIGEL™ prior to study. Cells were dissociated from the surface for passage by 1mg/ml dispase dissociation. AFD1 cells were then seeded onto untreated wells of surface modified plate 4 (6-well format). In parallel, AFD1 cells were also plated onto Nunclon Delta™ plates treated with 1:30 dilution of growth factor reduced MATRIGEL™ to provide as positive controls. In all treatments cells were maintained in MEF conditioned media.

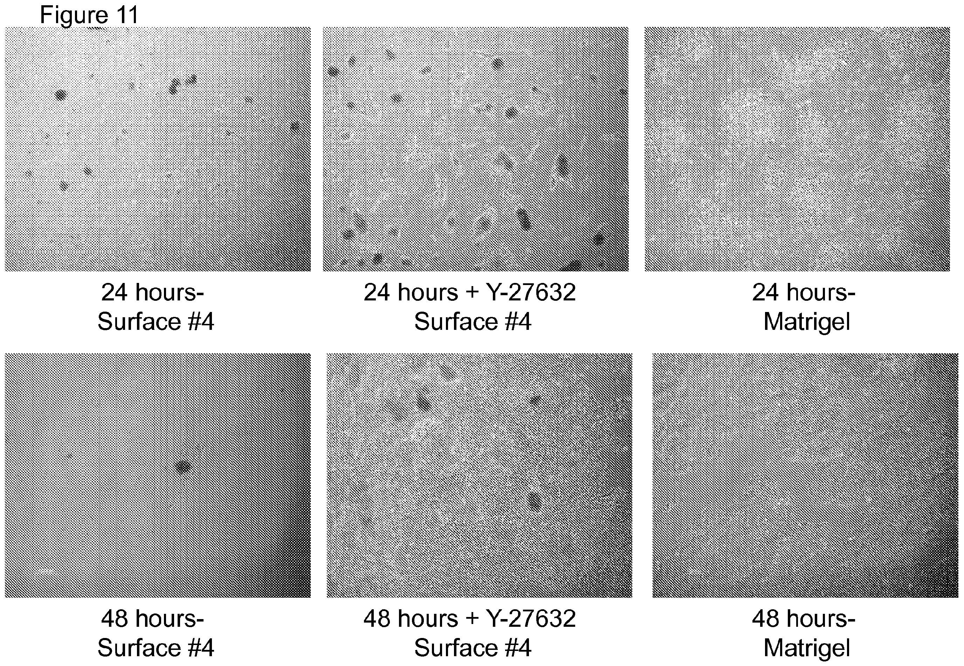

AFD1 cells seeded onto surface modified plate 4 did attach, however the attachment efficiency was much lower than on control plates (Figure 11). The attachment efficiency was greatly increased by the addition of a Rho Kinase inhibitor to the media. Adding either Y-27632 at a 10µM concentration or H1152-glycyl at a 3µM concentration for the first 24 hours in culture significantly increased plating efficiency of the cells to a rate similar observed with control plates (Figure 11). Cells were thereafter maintained in a constant 10µM Y-27632 concentration or a reduced 1µM concentration of H1152-glycyl, respectively, with daily media change.

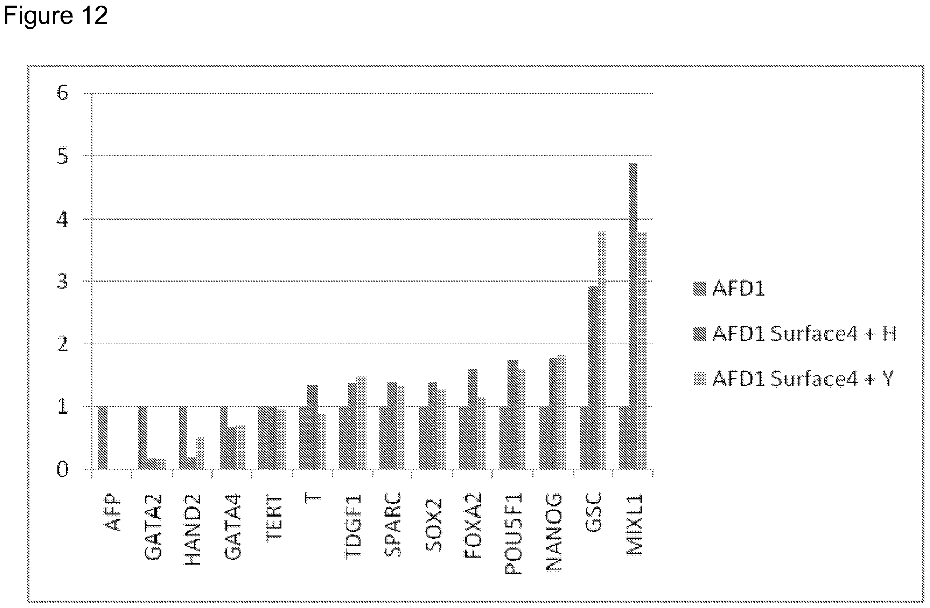

Cells were passaged twice on surface modified plate 4 in the presence of either Y-27632 or H1152-glycyl and were tested for the expression of genes associated with pluripotency by qRT-PCR (Figure 12). Expression of genes associated with and required for pluripotency (OCT4, NANOG, SOX2, TERT, and CRIPTO/TDGF) was maintained in AFD1 cells grown on modified surface 4 versus cells grown on control plates. It was also observed that the relative expression of several genes (AFP, HAND2, and GATA2) associated with spontaneous differentiation to extra-embryonic ectoderm or a trophoblast fate was decreased significantly. This may have been due to increased expression of NANOG, which inhibits differentiation by suppressing expression of extra-embryonic ectoderm or trophoblast associated genes. Additionally, this decrease in expression of extra-embryonic ectoderm or trophoblast associated genes could occur through differential adhesion and proliferation of cells more pluripotent, and less differentiated, on modified surface 4 versus control plates.

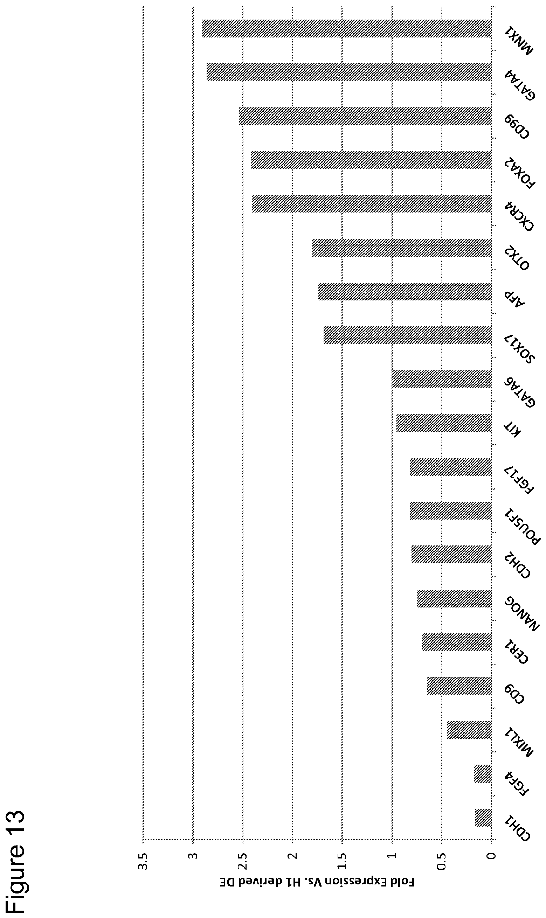

The pluripotency of cells grown on modified surface 4 was also confirmed by testing their capacity to differentiate to definitive endoderm. It was observed that cells differentiated to DE after growing several passages on modified surface 4 in MEF Conditioned Culture Media supplemented with Rho Kinase Inhibitor showed robust expression of genes associated with DE differentiation, comparable to expression levels and patterns observed with H1 hES differentiation to DE (Figure 13). Additionally, when assayed by flow cytometry, AFD1 cells grown for two passages with Rho Kinase inhibitor and differentiated on modified surface 4 were 63% positive for expression of CXCR4, a surface protein that correlates with definitive endoderm formation, and this level of expression was consistent with expression levels observed in H1 hES cells differentiated to DE (60% CXCR4 positive; Table 2).

**Table 1: Summary of the Transduction Conditions used in the Present Invention**

| Condition # | Cell | Feeder/Matrigel | 3 compounds* | ROCK inhibitor | Polybrene | PSC | Transduction Ratio (vol) |
|---|---|---|---|---|---|---|---|
| 1 | CRL2522 | Feeder | no | yes | Yes^ | + | 1:1:1:1 |
| 2 | CRL2522 | Feeder | no | yes | Yes^ | - | 5:1:1:1 |
| 3 | AF | Feeder | no | no | no | - | 10:1:1:1 |
| 4 | CRL2429 | Feeder | no | no | no | - | 10:1:1:1 |
| 5 | AF | Feeder | yes | yes | no | - | 10:1:1:1 |
| 6 | CRL2429 | Feeder | yes | yes | no | - | 10:1:1:1 |
| 7 | AF | Matrigel | no | yes | no | + | 10:1:1:1 |
| | | ^2.5µl Polybrene per 2.5ml media | | | | | Total volume to 2.5ml w/ EMEM media |
| | | | hOCT4 | hSOX2 | hNANOG | hLIN28 | |
| | | Transduction Ratio | | | | | |
| | | 1:1:1:1 | 0.1ml | 0.1ml | 0.1ml | 0.1ml | |
| | | Transduction Ratio | | | | | |
| | | 5:1:1:1 | 0.5ml | 0.1ml | 0.1ml | 0.1ml | |
| | | Transduction Ratio | | | | | |
| | | 10:1:1:1 | 0.5ml | 0.05ml | 0.05ml | 0.05ml | |
| | | | | | | | |

| | | Compounds* | Conc. | | | | |
|---|---|---|---|---|---|---|---|
| | | CHIR99021 | 100nM | | | | |
| | | BIX01294 | 1µM | | | | |
| | | R(T)Bay K 8644 | 2µM | | | | |

**Table 2: Expression of Markers Characteristic of the Definitive Endoderm Lineage Following the Differentiation of Pluripotent Stem Cells Derived From CRL2522 Cells**

| **#Clone** | **% Positive CXCR4** | **Normalized % 0f H1** |
|---|---|---|
| **CL-2** | 40.2 | 67.00 |
| **CL-3** | 23.4 | 39.00 |
| **CL-4** | 39.1 | 65.17 |
| **CL-5** | 32.2 | 53.67 |
| **CL-6** | 55.9 | 93.17 |
| **CL-7** | 48.2 | 80.33 |
| **CL-10** | 58.3 | 97.17 |
| **CL-12** | 53.6 | 89.33 |
| H1 hES | 60 | 100.00 |

**Table 3: Expression of Markers Characteristic of the Pancreatic Endocrine Lineage Following the Differentiation of Pluripotent Stem Cells Derived From CRL2522 and Amniotic Fluid-Derived Cells**

| | SST | PDX1 | PAX6 | PAX4 | NKX6.1 | NEUROD | INSULIN | GLUCAGON |
|---|---|---|---|---|---|---|---|---|
| CL-3 | 142 | 4 | 112 | 0.0 | 132 | 64 | 1 | 109 |
| CL-4 | 182 | 6 | 187 | 0.7 | 198 | 74 | 129 | 59 |
| CL-5 | 133 | 10 | 212 | 0.0 | 190 | 73 | 1000 | 263 |
| Mixed Clones (N>50) | 185 | 100 | 122 | 3.5 | 162 | 108 | 15566 | 3072 |
| AFD1 | 125 | 179 | 83 | 2 | 632 | 218 | 1413 | 2312 |
| AFD2 | 110 | 307 | 20 | 12 | 62 | 165 | 2499 | 149 |
| H1 differentiated | 8300 | 4164 | 455 | 635.3 | 842 | 11824 | 27540234 | 77671635 |
| H1hES | 1 | 1 | 1 | 1.0 | 1 | 1 | 1 | 1 |

## Claims

1. A method to produce pluripotent stem cells from human amniotic fluid-derived cells without the use of a feeder-cell layer and without the use of an agent that increases efficiency of retroviral transfection comprising;
a) plating human amniotic fluid-derived cells on a tissue culture substrate;
b) transducing the plated amniotic fluid-derived cells with at least one retrovirus containing nucleic acid encoding SOX2, OCT4, LIN28, and NANOG;
c) culturing the transduced cells, then transferring the transduced cells onto tissue culture plates coated with an extracellular matrix protein, and culturing the transferred cells in medium supplemented with an agent that inhibits Rho kinase activity, and bFGF, for one week; and
d) passaging the cultured cells onto tissue culture plates coated with an extracellular matrix protein and culturing the passaged cells in conditioned medium containing soluble factors derived from feeder cells and supplemented with bFGF, wherein the cells are transduced to introduce SOX2, OCT4, LIN28 and NANOG into the cells.

2. The method of claim 1, wherein the retrovirus is a lentivirus.

3. The method of claim 1 or claim 2, wherein the conditioned medium is conditioned using mouse embryonic fibroblasts.

4. The method of any one of claims 1 to 3, wherein the agent that inhibits Rho kinase activity is selected from the group consisting of Y-27632, Fasudil, (S)-(+)-4-Glycyl-2-methyl-1-[(4-methyl-5-isoquinolinyl) sulfonyl]-hexahydro-1H-1,4-diazepine dihydrochloride (referred to herein as H1152-glycyl) and Hydroxyfasudil.

5. The method of any one of claims 1 to 4, wherein the extracellular matrix comprises one or more component(s) from the group consisting of: fibronectin, vitronectin, laminin, collagen, gelatin, thrombospondin or MATRIGEL.

6. The method of claim 5, wherein the extracellular matrix is MATRIGEL.

7. A method to produce cells which are differentiated, for example cells expressing markers characteristic of the definitive endoderm lineage and/or cells expressing markers characteristic of the pancreatic endoderm lineage and/or cells expressing markers characteristic of the pancreatic endocrine lineage using a method comprising the method according to any one of claims 1 to 6.

## Patentansprüche

1. Verfahren zum Produzieren pluripotenter Stammzellen aus von menschlicher Amnionflüssigkeit stammenden Zellen ohne die Verwendung einer Feeder-Zellschicht und ohne die Verwendung eines Mittels, das die Effizienz retroviraler Transfektion steigert, umfassend:
a) Ausplattieren der von menschlicher Amnionflüssigkeit stammenden Zellen auf einem Gewebekultursubstrat;
b) Transduzieren der ausplattierten von Amnionflüssigkeit stammenden Zellen mit mindestens einem Retrovirus, das Nukleinsäure enthält, die SOX2, OCT4, LIN28 und NANOG codiert;
c) Züchten der transduzierten Zellen, dann Überführen der transduzierten Zellen auf Gewebekulturplatten, die mit einem extrazellulären Matrixprotein beschichtet sind, und Züchten der überführten Zellen in Medium, das mit einem die Rho-Kinaseaktivität hemmenden Mittel und bFGF angereichert ist, für eine Woche; und
d) Passagieren der gezüchteten Zellen auf Gewebekulturplatten, die mit einem extrazellulären Matrixprotein beschichtet sind und Züchten der passagierten Zellen in konditioniertem Medium, das lösliche Faktoren enthält, die von Feeder-Zellen stammen, und mit bFGF angereicht ist, wobei die Zellen derart transduziert sind, dass sie SOX2, OCT4, LIN28 und NANOG in die Zellen einbringen.

2. Verfahren nach Anspruch 1, wobei das Retrovirus ein Lentivirus ist.

3. Verfahren nach Anspruch 1 oder Anspruch 2, wobei das konditionierte Medium mit Hilfe embryonaler Fibroblasten aus Maus konditioniert wird.

4. Verfahren nach einem der Ansprüche 1 bis 3, wobei das die Rho-Kinaseaktivität hemmende Mittel ausgewählt ist aus der Gruppe, bestehend aus: Y-27632, Fasudil, (S)-(+)-4-Glycyl-2-methyl-1-[(methyl-5-isochinolinyl)sulfonyl]-hexahydro-1H-1,4-diazepin-Dihydrochlorid (hierin als H1152-Glycyl bezeichnet) und Hydroxyfasudil.

5. Verfahren nach einem der Ansprüche 1 bis 4, wobei die extrazelluläre Matrix eine oder mehrere Komponente(n) aus der Gruppe umfasst, bestehend aus: Fibronectin, Vitronectin, Laminin, Kollagen, Gelatine, Thrombospondin oder MATRIGEL.

6. Verfahren nach Anspruch 5, wobei die extrazelluläre Matrix MATRIGEL ist.

7. Verfahren zum Produzieren von Zellen, die differenziert sind, beispielsweise Zellen, die Marker exprimieren, die für die endgültige Endodermlinie charakteristisch sind, und/oder Zellen, die Marker exprimieren, die für die Pankreas-Endodermlinie charakteristisch sind und/oder Zellen, die Marker exprimieren, die für die endokrine Pankreaslinie charakteristisch sind, mit Hilfe eines Verfahrens, das das Verfahren nach einem der Ansprüche 1 bis 6 umfasst.

## Revendications

1. Procédé de production de cellules souches pluripotentes à partir de cellules dérivées de fluide amniotique humain sans l'utilisation d'une couche de cellules nourricières et sans l'utilisation d'un agent qui augmente l'efficacité de transfection rétrovirale comprenant :
a) l'étalement sur plaque de cellules dérivées de fluide amniotique humain sur un substrat de culture de tissu ;
b) la transduction des cellules dérivées de fluide amniotique humain étalées sur plaque avec au moins un rétrovirus contenant un acide nucléique codant pour SOX2, OCT4, LIN28 et NANOG ;
c) la culture des cellules transduites, puis le transfert des cellules transduites sur des plaques de culture de tissu enduites avec une protéine de matrice extracellulaire, et la culture des cellules transférées dans un milieu supplémenté avec un agent qui inhibe l'activité Rho kinase, et bFGF, pendant une semaine ; et
d) le passage des cellules cultivées sur des plaques de culture de tissu enduites avec une protéine de matrice extracellulaire et la culture des cellules soumises à passage dans du milieu conditionné contenant des facteurs solubles dérivés de cellules nourricières et supplémentées avec bFGF, les cellules étant transduites pour introduire SOX2, OCT4, LIN28 et NANOG dans les cellules.

2. Procédé de la revendication 1, dans lequel le rétrovirus est un lentivirus.

3. Procédé de la revendication 1 ou la revendication 2, dans lequel le milieu conditionné est conditionné au moyen de fibroblastes embryonnaires de souris.

4. Procédé de l'une quelconque des revendications 1 à 3, dans lequel l'agent qui inhibe l'activité Rho kinase est choisi dans le groupe constitué des : Y-27632, fasudil, dichlorhydrate de (S)-(+)-4-glycyl-2-méthyl-1-[(4-méthyl-5-isoquinoléinyl)sulfonyl]-hexahydro-1H-1,4-diazépine (appelé présentement HI152-glycyle) et hydroxyfasudil.

5. Procédé de l'une quelconque des revendications 1 à 4, dans lequel la matrice extracellulaire comprend un ou plusieurs composant(s) dans le groupe constitué des : fibronectine, vitronectine, laminine, collagène, gélatine, thrombospondine ou MATRIGEL.

6. Procédé de la revendication 5, dans lequel la matrice extracellulaire est MATRIGEL.

7. Procédé de production de cellules qui sont différenciées, par exemple des cellules exprimant des marqueurs caractéristiques du lignage endodermique définitif et/ou des cellules exprimant des marqueurs caractéristiques du lignage endodermique pancréatique et/ou des cellules exprimant des marqueurs caractéristique du lignage endocrine pancréatique en utilisant un procédé comprenant le procédé selon l'une quelconque des revendications 1 à 6.
